(19) Europäisches Patentamt
European Patent Office
Office européen des brevets

(11) EP 4 143 342 B1

(12) EUROPEAN PATENT SPECIFICATION

(45) Date of publication and mention of the grant of the patent:
**10.06.2026 Bulletin 2026/24**

(21) Application number: **21723392.3**

(22) Date of filing: **28.04.2021**

(51) International Patent Classification (IPC):
***C12Q 1/6883*** *(2018.01)*

(52) Cooperative Patent Classification (CPC):
**C12Q 1/6883;** C12Q 2600/136; C12Q 2600/158

(86) International application number:
**PCT/IB2021/053516**

(87) International publication number:
**WO 2021/220182 (04.11.2021 Gazette 2021/44)**

(54) **METHODS OF IDENTIFYING MODULATORS OF THE IL-17 PATHWAY**
VERFAHREN ZUR IDENTIFIZIERUNG VON MODULATOREN DES IL-17-WEGS
PROCÉDÉS D'IDENTIFICATION DE MODULATEURS DE LA VOIE DE L'IL-17

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2020 US 202063017960 P**

(43) Date of publication of application:
**08.03.2023 Bulletin 2023/10**

(73) Proprietor: **Janssen Pharmaceutica NV**
**2340 Beerse (BE)**

(72) Inventors:
- **BLAIN, Katherine Y.**
  **San Diego, California 92121-1126 (US)**
- **DE LEON-TABALDO, Aimee Rose**
  **San Diego, California 92121-1126 (US)**
- **FOURIE, Anne M.**
  **San Diego, California 92121-1126 (US)**
- **LIU, Xuejun**
  **San Diego, California 92121-1126 (US)**
- **LUNA-ROMAN, Rosa**
  **San Diego, California 92121-1126 (US)**
- **RODRIGUEZ, JR., Michael Angelo**
  **San Diego, California 92121-1126 (US)**
- **GOLDBERG, Steven**
  **San Diego, California 92121-1126 (US)**
- **XUE, Xiaohua**
  **San Diego, California 92121 (US)**

(74) Representative: **Carpmaels & Ransford LLP**
**One Southampton Row**
**London WC1B 5HA (GB)**

(56) References cited:
**EP-A2- 2 288 382**
**EP-B1- 2 288 382**
**WO-A1-2016/044189**
**WO-A2-2009/082624**
**US-A1- 2010 322 897**
**US-A1- 2016 159 914**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 143 342 B1

## Description

CROSS-REFERENCE TO RELATED APPLICATIONS

This application claims the benefit under 35 U.S.C. § 119(e) of U.S. Provisional Application Number 63/017,960, filed April 30, 2020.

FIELD

The invention relates to methods, and kits for identifying agents that modulate the IL-17 pathway.

BACKGROUND

Interleukin-17 (IL-17) is a cytokine secreted by activated T helper 17 (Th17) cells, CD8+ T cells, γδ T cells, ILC3 and NK cells in response to cytokines such as IL-23, IL-1β and TGF-β. IL-17 regulates production of mediators such as antimicrobial peptides, proinflammatory cytokines and chemokines from multiple cell types including fibroblasts and synoviocytes that are involved in the recruitment of neutrophils and pathology of tissue damage in inflammation or in host defense. IL-17 also synergizes with other cytokines, such as TNF-α and IL-Iβ to potentiate the pro-inflammatory environment.

Because of its involvement in immune regulatory functions, inhibitors of IL-17 are being investigated as possible treatments for autoimmune diseases such as psoriasis, psoriatic arthritis, ankylosing spondylitis, and multiple sclerosis. Psoriasis is a common, chronic inflammatory skin disease with a complex etiology involving genetic risk factors and environmental triggers. Psoriasis arises through chronic interactions between hyper-proliferative keratinocytes and infiltrating, activated immune cells (Harden J. et al. J Autoimmun. 2015; 64: 66-73). IL-17 is a proinflammatory cytokine and prominent mediator downstream of interleukin-23 (IL-23) in psoriasis. IL-17 acts directly on keratinocytes to elicit an inflammatory response, and antibodies that neutralize IL-17 or block its receptor have become highly effective medicines for psoriasis patients (Ha H.-L. et al. Proc. Natl. Acad. Sci. U.S.A. 2014; 111(33): E3422-3431 While no oral small molecule IL-17 pathway antagonists have progressed into late stage clinical trials yet, they remain an attractive area for discovery as their development can broaden treatment options for many patients without access to biologics. Therefore, identification of a small molecule inhibiting the IL-17 pathway would provide therapeutic opportunity for psoriasis, as well as other IL-17 driven autoimmune diseases.

US 2016/159914 relates to antagonists of the IL-17RA-IL-17RE heteromeric receptor complex.

EP 2 288 382 A2 relates to human antibodies that recognize the heterodimeric IL-17A/ IL-17F complex.

BRIEF SUMMARY

Accordingly, there is a need to develop methods to identify inhibitors of the IL-17 pathway. The present invention satisfies this need by providing kits and methods for identifying an agent that modulates the IL-17 pathway. The present invention is defined by the appended claims.

In a general aspect, disclosed herein is a method of identifying an agent that modulates the IL-17 pathway.

In some embodiments, the method comprises:

a) contacting a cell with IL-17, preferably IL-17A, or T cell conditioned media;
b) contacting the cell with the agent;
c) incubating the cell with the IL-17 or T cell conditioned media, and the agent;
d) collecting the cell supernatant after the incubating step;
e) detecting a level of granulocyte colony-stimulating factor (G-CSF) in the cell supernatant; and
f) comparing the level of G-CSF in the cell supernatant contacted with the agent with a control cell supernatant, wherein a modulation in the level of G-CSF in the cell supernatant contacted with the agent as compared to the control cell supernatant indicates that the agent is capable of modulating the IL-17 pathway.

In certain embodiments, the cell supernatant is collected 24 hours after contacting the cell with the agent.

In certain embodiments, the method further comprises:

a) collecting the cell;
b) detecting an expression level of one gene or a panel of genes of the cell selected from the group consisting of DEFB4A, S100A7A, SAA2, CCL20, RNASE7, SAA4, IL-23A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2, CSF3,

LCN2, SPRR3, DHRS9, GLUL, RHCD, CXCL2, SERPINB3, and S100P, after the incubating step;
c) comparing the expression level of the one gene or panel of genes of the cell contacted with the agent with a control cell, wherein a modulation in the expression level of the one or more gene(s) in the cell contacted with the agent as compared to the control cell verifies that the agent is capable of modulating the IL-17 pathway.

In another embodiment, the method comprises:

a) contacting a cell with IL-17, preferably IL-17A, or T cell conditioned media;
b) contacting the cell with the agent;
c) incubating the cell with the IL-17 or T cell conditioned media, and the agent, preferably for 6 to 72 hours;
d) collecting the cell after the incubating step;
e) detecting an expression level of one gene or a panel comprising 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more genes of the cell selected from the group consisting of DEFB4A, S100A7A, SAA2, CCL20, RNASE7, SAA4, IL-23A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2, CSF3, LCN2, SPRR3, DHRS9, GLUL, RHCD, CXCL2, SERPINB3, and S100P, after the incubating step; and
f) comparing the expression level of the 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more genes of the cell contacted with the agent with a control cell, wherein a modulation in the expression level of the one or more gene(s) of the cell contacted with the agent as compared to the control cell indicates that the agent is capable of modulating the IL-17 pathway.

In certain embodiments, the agent is selected from the group consisting of a small molecule, a polypeptide, an antibody, and a polynucleotide.
In certain embodiments, the agent has an IC50 of 1 μM or less.
In certain embodiments, the agent is a small molecule modulator, such as an IL-17A inhibitor, preferably an IL-17Ai-1 to 6, selected from the group consisting of the following structures and pharmaceutically acceptable salts thereof:

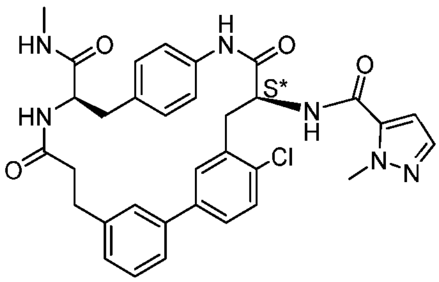

(IL-17Ai-1)

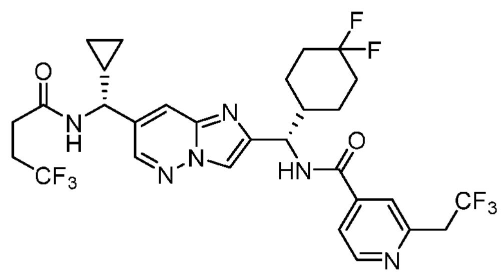

(IL-17Ai-2)

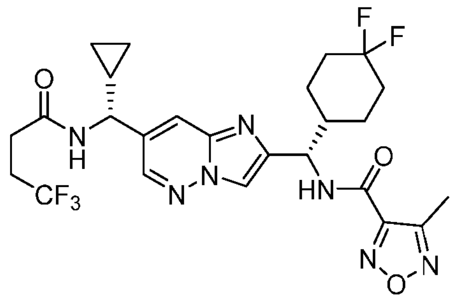

(IL-17Ai-3)

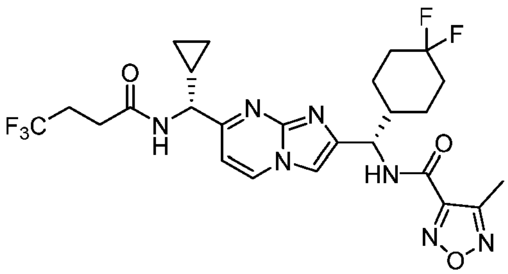

(IL-17Ai-4)

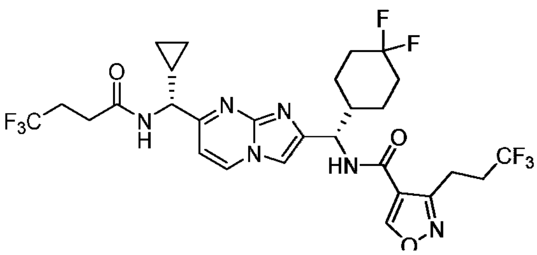

(IL-17Ai-5)

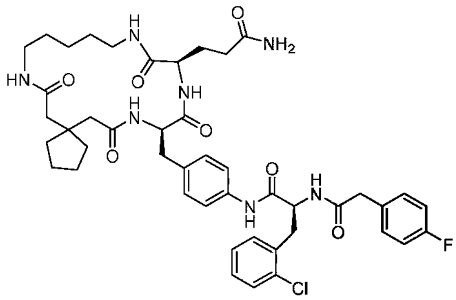

(IL-17Ai-6).

In certain embodiments, the cell is a keratinocyte or a fibroblast cell, preferably a human keratinocyte.

In another aspect, the disclosure provides a kit for identifying a modulator of the IL-17 pathway, the kit comprising

a) a set of probes capable of detecting a panel of genes comprising 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more genes selected from the group consisting of DEFB4A, S100A7A, SAA2, CCL20, RNASE7, SAA4, IL-23A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2, CSF3, LCN2, SPRR3, DHRS9, GLUL, RHCD, CXCL2, SERPINB3, and S100P; and

b) instructions for performing an assay to identify a modulator of the IL-17 pathway.

In certain embodiments, the panel of genes consists of DEFB4A, S100A7A, SAA2, CCL20, RNASE7, SAA4, IL-23A, S100A12, DEFB103A, C15orf48, ZC3H12A, SAA1, SPRR2B, PRSS22, CXCL1, IL-36G, NFKBIZ, IL-8, PLAT,

CXCL5, PDZK1IP1, RSAD2, KRT78, SLC39A2, and CSF3.

In certain embodiments, the probes are oligonucleotides.

Other aspects, features and advantages of the invention will be apparent from the following disclosure, including the detailed description of the invention and its preferred embodiments and the appended claims.

## BRIEF DESCRIPTION OF THE DRAWINGS

The foregoing summary, as well as the following detailed description of preferred embodiments of the present application, will be better understood when read in conjunction with the appended drawings. It should be understood, however, that the application is not limited to the precise embodiments shown in the drawings.

FIG. 1 shows the concentration of human IL-17A in media of unstimulated and undifferentiated CD4+ T cells and in conditioned media from Th17 cells.

FIGs. 2A-C show results of recombinant human IL-17 (rhIL-17A) stimulation in the absence and/or presence of inhibitor compounds in normal human keratinocytes (NHKs). FIGs. 2A and 2C show levels of granulocyte colony stimulating factor (G-CSF) (pg/ml) in the supernatant of NHK cells. FIG. 2B shows percent viability of NHK cells.

FIGs. 3A-P show G-CSF levels and cell viability of NHK cells in response to rhIL-17A stimulation and various doses of inhibitor compounds. FIGs. 3A-B: p38 inhibitor (p38i), FIGs. 3C-D: PAK inhibitor, FIGs. 3E-F: IL-17A inhibitor 1 (IL-17Ai-1), FIGs. 3G-H: IL-17A inhibitor 2 (IL-17Ai-2), FIGs. 3I-J: IL-17A inhibitor 3 (IL-17Ai-3), FIGs. 3K-L: IL-17A inhibitor 4 (IL-17Ai-4), FIGs. 3M-N: IL-17A inhibitor 5 (IL-17Ai-5), and FIGs. 3O-P: IL-17A inhibitor 6 (IL-17Ai-6).

FIG. 4A-B show results of Th17 cell conditioned media stimulation in the absence and/or presence of inhibitor compounds in normal human keratinocytes (NHKs). FIG. 4A shows G-CSF levels (pg/ml) in the supernatant. FIG. 4B shows dose-dependent inhibition of Th17 stimulated G-CSF production from NHKs by inhibitors.

FIGs. 5A-B show gene expression of a panel of genes from normal human keratinocytes (NHK) with or without rhIL-17 stimulation in the absence and/or presence of inhibitors.

FIG. 6 shows gene expression of a panel of genes from normal human keratinocytes (NHK) treated with standard tissue culture media or Th17 cell conditioned media in the absence and/or presence of inhibitors.

## DETAILED DESCRIPTION

Various publications, articles and patents are cited or described in the background and throughout the specification. Discussion of documents, acts, materials, devices, articles or the like which has been included in the present specification is for the purpose of providing context for the invention. Such discussion is not an admission that any or all of these matters form part of the prior art with respect to any inventions disclosed or claimed.

Unless defined otherwise, all technical and scientific terms used herein have the same meaning as commonly understood to one of ordinary skill in the art to which this invention pertains. Otherwise, certain terms used herein have the meanings as set forth in the specification.

It must be noted that as used herein and in the appended claims, the singular forms "a," "an," and "the" include plural reference unless the context clearly dictates otherwise.

Unless otherwise stated, any numerical values, such as a concentration or a concentration range described herein, are to be understood as being modified in all instances by the term "about." Thus, a numerical value typically includes $\pm$ 10% of the recited value. For example, a concentration of 1 mg/mL includes 0.9 mg/mL to 1.1 mg/mL. Likewise, a concentration range of 1% to 10% (w/v) includes 0.9% (w/v) to 11% (w/v). As used herein, the use of a numerical range expressly includes all possible subranges, all individual numerical values within that range, including integers within such ranges and fractions of the values unless the context clearly indicates otherwise.

Unless otherwise indicated, the term "at least" preceding a series of elements is to be understood to refer to every element in the series. Those skilled in the art will recognize, or be able to ascertain using no more than routine experimentation, many equivalents to the specific embodiments described herein.

As used herein, the terms "comprises," "comprising," "includes," "including," "has," "having," "contains" or "containing," or any other variation thereof, will be understood to imply the inclusion of a stated integer or group of integers but not the exclusion of any other integer or group of integers and are intended to be non-exclusive or open-ended. For example, a composition, a mixture, a process, a method, an article, or an apparatus that comprises a list of elements is not necessarily limited to only those elements but can include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

It should also be understood that the terms "about," "approximately," "generally," "substantially" and like terms,

used herein when referring to a dimension or characteristic of a component of the preferred invention, indicate that the described dimension/ characteristic is not a strict boundary or parameter and does not exclude minor variations therefrom that are functionally the same or similar, as would be understood by one having ordinary skill in the art. At a minimum, such references that include a numerical parameter would include variations that, using mathematical and industrial principles accepted in the art (e.g., rounding, measurement or other systematic errors, manufacturing tolerances, etc.), would not vary the least significant digit.

As used herein, the term "polynucleotide," synonymously referred to as "nucleic acid molecule," "nucleotides" or "nucleic acids," refers to any polyribonucleotide or polydeoxyribonucleotide, which can be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that can be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications can be made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short nucleic acid chains, often referred to as oligonucleotides.

As used herein, the terms "peptide," "polypeptide," or "protein" can refer to a molecule comprised of amino acids and can be recognized as a protein by those of skill in the art. The conventional one-letter or three-letter code for amino acid residues is used herein. The terms "peptide," "polypeptide," and "protein" can be used interchangeably herein to refer to polymers of amino acids of any length. The polymer can be linear or branched, it can comprise modified amino acids, and it can be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

The term "expression" as used herein, refers to the biosynthesis of a gene product. The term encompasses the transcription of a gene into RNA. The term also encompasses translation of RNA into one or more polypeptides, and further encompasses all naturally occurring post-transcriptional and post-translational modifications. The expressed RNA or polypeptide can be within the cytoplasm of a cell, into the extracellular milieu such as the growth medium of a cell culture or anchored to the cell membrane.

The term "level of gene expression" or "expression level" refers to the level (e.g., amount) of one or more products (e.g. RNA, protein) encoded by a given gene in a sample or reference standard. The expression level can be relative or absolute.

As used herein, the term "normalize" or "normalizing" refers to a manipulation of discrete expression level data wherein the expression level of one or more test genes is expressed relative to the expression level of one or more control genes, such as one or more housekeeping genes, or the expression of the same gene or genes in a control cell. For example, numerical expression level value one or more housekeeping genes can be deducted from the numerical expression level value of one or more test genes thereby permitting comparison of normalized marker values among a plurality of samples or to a reference.

The term "housekeeping gene" as used herein, refers to a gene encoding a transcript and/or protein that is constitutively expressed and is necessary for basic maintenance and essential cellular functions. A housekeeping gene generally is not expressed in a cell- or tissue- dependent manner, most often being expressed by all cells in a given organism. Some examples of housekeeping proteins include B2M, TFRC, YWHAZ, RPLO, 18S, GUSB, UBC, TBP, GAPDH, PPIB, POLR2A, ACTB, PGK1, HPRT1, IPO8 or HMBS, among others.

In an attempt to help the reader of the application, the description has been separated in various paragraphs or sections, or is directed to various embodiments of the application. These separations should not be considered as disconnecting the substance of a paragraph or section or embodiments from the substance of another paragraph or section or embodiments. To the contrary, one skilled in the art will understand that the description has broad application and encompasses all the combinations of the various sections, paragraphs and sentences that can be contemplated. The discussion of any embodiment is meant only to be exemplary and is not intended to suggest that the scope of the disclosure, is limited to these examples.

As used herein, "IL-17" refers to interleukin 17, including all family members, IL-17A, IL-17B, IL-17C, IL-17D, IL-17E and IL-17F. IL-17 is also named IL17, CTLA8, and CTLA-8. IL-17 is a pro-inflammatory cytokine produced by a group of T helper cell known as Th17 cell in response to stimulation with IL-23. All members of the IL-17 family have a similar protein structure. IL-17 regulates the activities of NF-kappaB and mitogen-activated protein kinases. High levels of IL-17 are associated with several chronic inflammatory diseases including psoriasis, psoriatic arthritis, ankylosing

spondylitis, rheumatoid arthritis, and multiple sclerosis. An "IL-17" includes an IL-17 from any animal species, as well as a recombinant product of IL-17. IL-17 could be a homodimer of IL-17 or a heterodimer of IL-17. An exemplary amino acid sequence of human IL-17 is represented in GenBank Accession No. NP_002181.1, which can be encoded by a nucleic acid sequence such as that of GenBank Accession No. NM_002190.3.

As used herein, "IL-17 pathway" refers to any component involved in IL-17 signaling and response, including, but not limited to IL-17, IL-17 receptor, and IL-17-induced genes and/or proteins.

The term "modulator" or "an agent that modulates" as used herein refers to any agents or molecules that can disrupt, prevent, inhibit, neutralize, antagonize and/or interfere with the expression, activity and/signaling of IL-17, IL-17 receptor or the IL-17 pathway. A modulator includes, but is not limited to, a small molecule, a polypeptide, an antibody, and a polynucleotide. Non-limiting examples of modulators are the anti-IL-17 antibody Secukinumab (Caligor Coghlan, Secaucus, NJ) and the anti-IL-17 receptor antibody Brodalumab (Caligor Coghlan, Secaucus, NJ).

As used herein, the term "small molecule" or "SM" refers to any compound that has a molecular weight ranging from about 100 g/mol to about 1500 g/mol. For example, a small molecule compound can have a molecular weight ranging from 400 g/mol to about 1050 g/mol, or from 500 g/mol to about 1000 g/mol, or from about 600 g/mol to about 900 g/mol, or from about 300 g/mol to about 750 g/mol, or from about 500 g/mol to about 800 g/mol. Alternatively, the SM modulators can have a molecular weight ranging from a lower limit of about 100 g/mol or 200 g/mol or about 300 g/mol or about 400 g/mol or about 500 g/mol or about 600 g/mol to a upper limit of about 400 g/mol or about 500 g/mol or about 600 g/mol or about 700 g/mol or about 800 g/mol or about 900 g/mol or about 1000 g/mol or about 1100 g/mol, or about 1200 g/mol or about 1300 g/mol or about 1400 g/mol or about 1500 g/mol. Examples of small molecule modulators of IL-17 include, but are not limited to, IL-17A inhibitors (IL-17Ai), such as IL-17Ai-1 to IL-17Ai-6 described herein, or pharmaceutically acceptable salts thereof.

For small molecules, the absolute stereochemistry is specified according to the Cahn-Ingold-Prelog R-S system. Chiral centers, of which the absolute configurations are known, are depicted or labelled by prefixes R and S, assigned by the standard sequence-rule procedure, and preceded when necessary by the appropriate locants (Pure & Appl. Chem. 45, 1976, 11-30). Certain examples contain chemical structures that are depicted or labelled as an (R*) or (S*). When (R*) or (S*) is used in the name of a compound or in the chemical representation of the compound, it is intended to convey that the compound is a pure single isomer at that stereocenter; however, absolute configuration of that stereocenter has not been established. Thus, a compound designated as (R*) refers to a compound that is a pure single isomer at that stereocenter with an absolute configuration of either (R) or (S), and a compound designated as (S*) refers to a compound that is a pure single isomer at that stereocenter with an absolute configuration of either (R) or (S). For example,

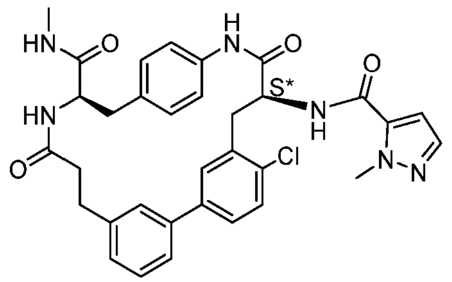

refers to a compound that is either:

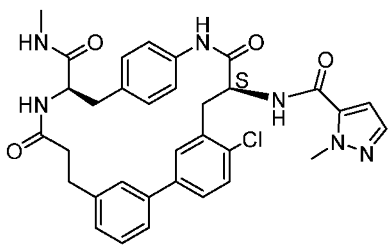

or

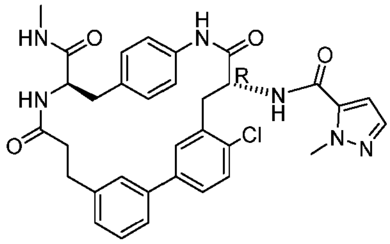

.

As used herein, the term "antibody" is used in a broad sense and includes immunoglobulin or antibody molecules including human, humanized, composite and chimeric antibodies and antibody fragments that are monoclonal or polyclonal. In general, antibodies are proteins or peptide chains that exhibit binding specificity to a specific antigen. The antibody can be derived from any species and can be IgM, IgG (e.g. IgGl, IgG2, IgG3, or IgG4), IgD, IgA, or IgE, for example.

As used herein, the term "antigen-binding fragment" refers to an antibody fragment such as, for example, a

diabody, a Fab, a Fab', a F(ab')2, an Fv fragment, a disulfide stabilized Fv fragment (dsFv), a $(dsFv)_2$, a bispecific dsFv (dsFv-dsFv'), a disulfide stabilized diabody (ds diabody), a single-chain antibody molecule (scFv), a single domain antibody (sdab) an scFv dimer (bivalent diabody), a multispecific antibody formed from a portion of an antibody comprising one or more CDRs, a camelized single domain antibody, a nanobody, a domain antibody, a bivalent domain antibody, or any other antibody fragment that binds to an antigen but does not comprise a complete antibody structure.

As used herein, "G-CSF" or "GCSF" refers to granulocyte colony-stimulating factor. It is also named CSF3 or C17orf33. Granulocyte/macrophage colony-stimulating factors are cytokines that act in hematopoiesis by controlling the production, differentiation, and function of 2 related white cell populations of the blood, the granulocytes and the monocytes-macrophages. IL-17 induces expression of G-CSF in stromal cells (Fossiez et al. (1996) J Exp Med. 183(6):2593-603).

The present invention relates generally to identifying an agent that modulates the IL-17 pathway, and provides methods, and kits useful for this purpose.

Methods

The methods of the invention are defined in the appended claims. According to one particular aspect, disclosed herein is a method of identifying an agent that modulates the interleukin-17 (IL-17) pathway, the method comprising:

a) contacting a cell with IL-17, preferably IL-17A, or T cell conditioned media;
b) contacting the cell with the agent;
c) incubating the cell with the IL-17 or the T cell conditioned media, and the agent;
d) collecting the cell supernatant after the incubating step;
e) detecting a level of granulocyte colony-stimulating factor (G-CSF) in the cell supernatant; and
f) comparing the level of G-CSF in the cell supernatant contacted with the agent with a control cell supernatant, wherein a modulation in the level of G-CSF in the cell supernatant contacted with the agent as compared to the control cell supernatant indicates that the agent is capable of modulating the IL-17 pathway.

In one embodiment, the cell supernatant is collected between 6 and 72 hours after contacting the cell with the agent. Preferably, the cell supernatant is collected 24 hours after contacting the cell with the agent.

Any methods available in the art for detecting a level of G-CSF are encompassed herein. For example, a level of G-CSF can be measured using an antibody. The antibody can be labeled (e.g., fluorescently) or unlabeled. Additionally, the antibody can be free in solution or immobilized.

In one embodiment, a level of G-CSF is measured using a homogeneous time-resolved fluorescence assay (HTRF). This technology combines fluorescence resonance energy transfer technology (FRET) with time-resolved measurement (TR). For example, G-CSF is detected in a sandwich assay format using different specific antibodies, one labeled with $Eu^{3+}$ - Cryptate (donor) and the second with d2 (acceptor). When the dyes are in close proximity, the excitation of the donor with a light source (laser or flash lamp) triggers a Fluorescence Resonance Energy Transfer (FRET) towards the acceptor, which in turn fluoresces at a specific wavelength. The two conjugates bind to the antigen present in the sample, thereby generating FRET. Signal intensity is proportional to the number of antigen-antibody complexes formed and therefore to the G-CSF concentration.

In certain embodiments, the agent decreases the level of G-CSF in the cell supernatant.

In one embodiment, the method further comprises a) collecting the cell; b) detecting an expression level of one gene or a panel of genes of the cell selected from the group consisting of DEFB4A, S100A7A, SAA2, CCL20, RNASE7, SAA4, IL-23A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2, CSF3, LCN2, SPRR3, DHRS9, GLUL, RHCD, CXCL2, SERPINB3, and S100P, after the incubating step; c) comparing the expression level of the one gene or panel of genes of the cell contacted with the agent with a control cell, wherein a modulation in the expression level of the one or more gene(s) in the cell contacted with the agent as compared to the control cell verifies that the agent is capable of modulating the IL-17 pathway.

Any methods available in the art for detecting expression of a gene or panel of genes are encompassed herein. In certain embodiments, genes are detected at the nucleic acid (e.g., RNA) level. For example, the amount of RNA (e.g., mRNA) present in a sample is measured (e.g., to determine the level of expression). Thus, "detecting expression" encompasses instances where a gene is determined not to be expressed, not to be detectably expressed, expressed at a low level, expressed at a normal level, or overexpressed. Nucleic acid (e.g., RNA, amplified cDNA, etc.) can be detected/quantified using a variety of nucleic acid techniques known to those of ordinary skill in the art, including but not limited to, nucleic acid hybridization and nucleic acid amplification. mRNA expression in a sample can be quantified using northern blotting or in situ hybridization, RNase protection assays, microarrays or PCR-based methods, such as reverse transcription polymerase chain reaction (RT-PCR) optionally followed by quantitative PCR (qPCR). RT-PCR step

is typically primed using specific primers, random hexamers, or oligo-dT primers, depending on the circumstances and the goal of expression profiling.

Level of gene expression can also be analyzed using microarrays or RNA sequencing (RNAseq) using commercially available platforms such as those from Luminex, Affymetrix, Illumina and Agilent. For example, QuantiGene Plex Gene Expression Assay (ThermoFisher; Waltham, MA) uses hybridization of target specific probe sets and then signal amplification is achieved using branch DNA technology with labeled probes. In a final step, addition of streptavidin phycoerythrin (SAPE) generates a signal that is proportional with the amount of target RNA present in the sample. The signal is read using a Luminex instrument.

As used herein, "probe" refers to any molecule or agent that is capable of selectively binding to an intended target biomolecule. The target molecule can be a gene, for example, a nucleotide transcript corresponding to a gene. Probes can be synthesized by one of skill in the art, or derived from appropriate biological preparations, in view of the present disclosure. Probes can be specifically designed to be labeled. Examples of molecules that can be utilized as probes include, but are not limited to, a nucleic acid (such as an oligonucleotide hybridizing to the gene or mRNA), proteins, peptides, antibodies, aptamers, affibodies, and organic molecules.

In another aspect, disclosed herein is a method of identifying an agent that modulates the interleukin-17 (IL-17) pathway, the method comprising:

a) contacting a cell with IL-17 or T cell conditioned media;
b) contacting the cell with the agent;
c) incubating the cell with the IL-17 or the T cell conditioned media, and the agent, preferably for 6 to 72 hours;
d) collecting the cell after the incubating step;
e) measuring an expression level of one gene or a panel comprising 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more genes of the cell selected from the group consisting of DEFB4A, S100A7A, SAA2, CCL20, RNASE7, SAA4, IL-23A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2, CSF3, LCN2, SPRR3, DHRS9, GLUL, RHCD, CXCL2, SERPINB3, and S100P, after the incubating step; and
f) comparing the expression level of the 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more genes of the cell contacted with the agent with a control cell, wherein a modulation in the expression level of the one or more gene(s) of the cell contacted with the agent as compared to the control cell indicates that the agent is capable of modulating the IL-17 pathway.

In certain embodiments, the methods comprise contacting the cell with IL-17 and the agent sequentially. In other embodiments, the method comprises contacting the cell with IL-17 and the agent concurrently. For example, IL-17 and the agent can be combined and incubated before contacting the cell.

In certain embodiments, the IL-17 is recombinant IL-17, preferably recombinant human IL-17; more preferably recombinant human IL-17A. In one embodiment, the methods comprise contacting the cell with 0.2 ng/ml to 200 ng/ml IL-17. Preferably, the method comprises contacting the cell with 5 ng/ml IL-17.

In certain embodiments, the T cell conditioned media is Th17 cell conditioned media. In certain embodiments the Th17 conditioned media comprises IL-17, preferably IL-17A.

In certain embodiments, the agent is selected from the group consisting of a small molecule, a polypeptide, an antibody, and a polynucleotide. Any concentration of the agent that maintains cell viability can be used in the method. The concentration of an agent to be used and cell viability can be measured using a variety of techniques known to those of ordinary skill in the art, such as those exemplified herein.

Exemplary small molecule modulators of IL-17 used herein include, without limitation, IL-17A inhibitors IL-17Ai-1 to IL-17Ai-6 listed in Table 1 below and pharmaceutically acceptable salts thereof:

Table 1. Exemplary Small Molecule Modulators of IL-17A

| Modulator | Structure | Reference |
|---|---|---|
| IL-17Ai-1 | 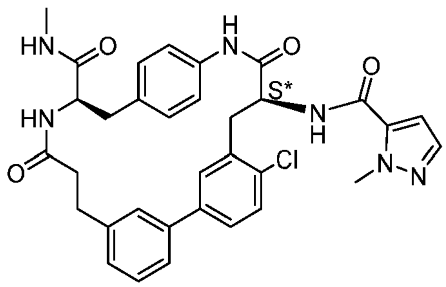 | IL-17Ai-1 is disclosed as a mixture of diastereomers in "Binding site elucidation and structure guided design of macrocyclic IL-17A antagonists," Scientific Reports, 6, DOI: 10.1038/srep30859 (2016) |

(continued)

| Modulator | Structure | Reference |
|---|---|---|
| IL-17Ai-2 | 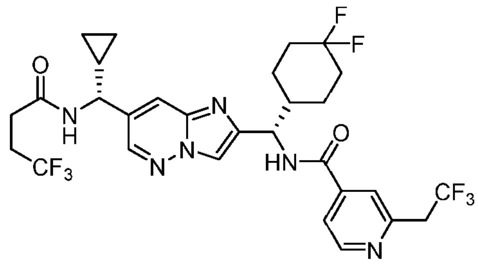 | US Application No. 63/017682 |
| IL-17Ai-3 | 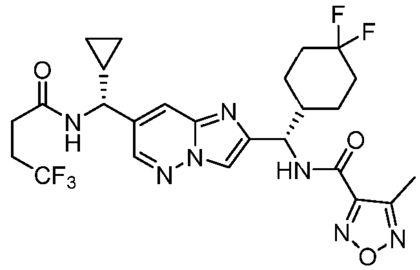 | US Application No. 63/017682 |
| IL-17Ai-4 | 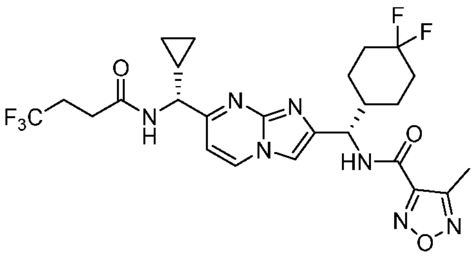 | US Application No. 63/017679 |
| IL-17Ai-5 | 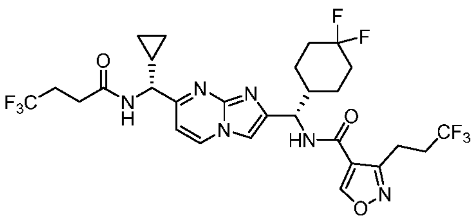 | US Application No. 63/017679 |
| IL-17Ai-6 | 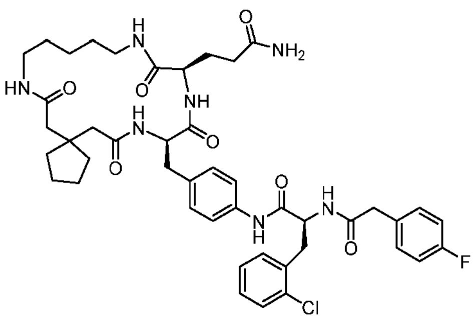 | Compound 479 in WO 2013/116682 |

The following compound:

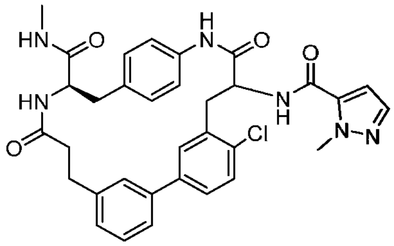

is disclosed in Liu, S. et al. "Binding site elucidation and structure guided design of macrocyclic IL-17A antagonists," Scientific Reports, 6 30859, DOI: 10.1038/srep30859 (2016). This compound was prepared as a mixture of diastereomers, which were separated by supercritical fluid chromatography (SFC) (column: DAICEL CHIRALPAK AD (250 mm * 30 mm, 10 um); mobile phase: [0.1% $NH_3H_2O$ IPA]; B%: 40% - 40%,5.3 min ; 420 min). The more potent diastereomer. assigned herein as IL-17Ai-1:

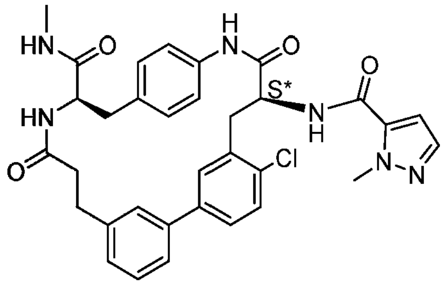

was evaluated using the method disclosed herein.

Modulator IL-17Ai-6 is disclosed in PCT Patent Application Publication No. WO 2013/116682, titled: "Macrocyclic Compounds for Modulating IL-17," (see e.g., compound 479).

In certain embodiments, the agent has an IC50 of 1 μM or less. The half maximal inhibitory concentration (IC50) is a measure of the effectiveness of a substance in inhibiting a specific biological or biochemical function. This quantitative measure indicates how much of a particular substance is needed to inhibit a given biological process by half, and is commonly used in the art. The IC50 can be determined with methods known in the art in view of the present disclosure. It can be determined with functional assays or with competition binding assays. In certain embodiments, the IC50 is the concentration of an agent required to inhibit, in vitro, IL-17-induced G-CSF secretion from cells by 50%.

In certain embodiments, the agent is a small molecule modulator selected from the group consisting of IL-17Ai-1 to 6 having the following structures and pharmaceutically acceptable salts thereof:

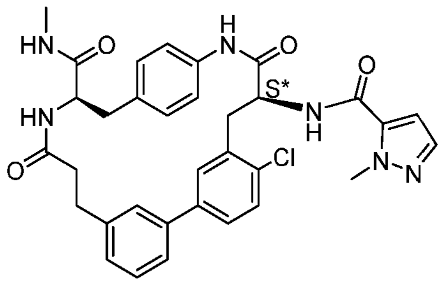

(IL-17Ai-1)

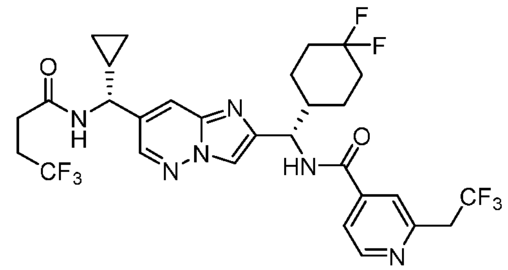

(IL-17Ai-2)

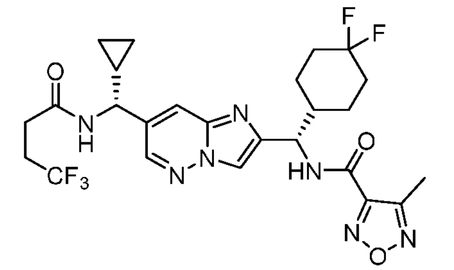

(IL-17Ai-3)

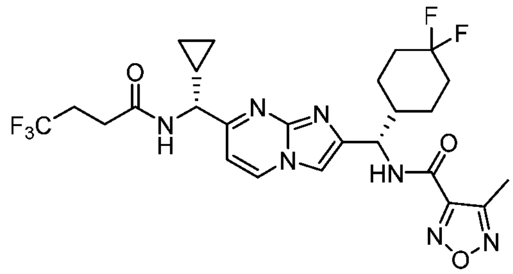

(IL-17Ai-4)

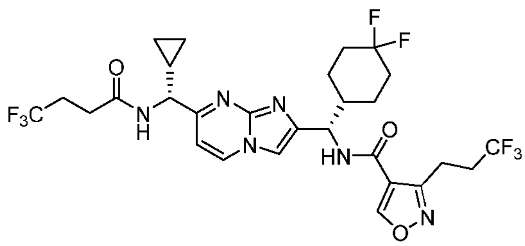

(IL-17Ai-5)

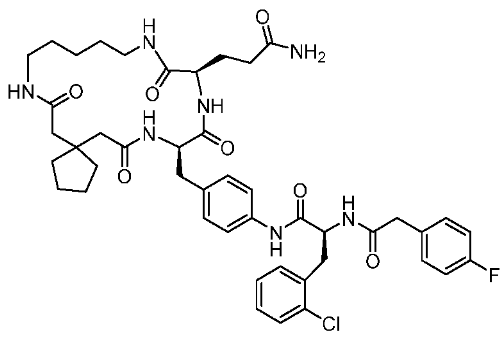

(IL-17Ai-6).

In one embodiment, the cell is incubated with the IL-17 and the agent for 6 to 72 hours in the incubating step. Preferably, the cell is incubated with the IL-17 and the agent for 24 hours in the incubating step.

In certain embodiments, the cell is a human cell. In one embodiment the cell is a fibroblast or a keratinocyte; preferably a human keratinocyte. In certain embodiments, the method further comprises growing the human keratinocyte in a keratinocyte growth medium until reaching about 70-90% confluence prior to contacting the cell with the IL-17.

In certain embodiments, the control cell is a cell contacted with IL-17 without the addition of compounds.

In certain embodiments, the panel of genes comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more genes of the cell selected from the group consisting of DEFB4A, S100A7A, SAA2, CCL20, RNASE7, SAA4, IL-23A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2, CSF3, LCN2, SPRR3, DHRS9, GLUL, RHCD, CXCL2, SERPINB3, and S100P. In one embodiment, the panel of genes consists of DEFB4A, S100A7A, SAA2, CCL20, RNASE7, SAA4, IL-23A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2, and CSF3.

## Kits

The kits of the invention are defined in the appended claims. In another aspect, the invention relates to kits for identifying a modulator of the IL-17 pathway.

The term "kit" as used herein refers to a combination of reagents and other materials. It is contemplated that the kit can include reagents such as buffering agents, protein stabilizing reagents, signal producing systems (e.g., florescence signal generating systems), antibodies, control proteins, as well as testing containers (e.g., microtiter plates, etc.). It is not intended that the term "kit" be limited to a particular combination of reagents and/or other materials. In one embodiment, the kit comprises instructions for use. The test kit can be packaged in any suitable manner, typically with the elements in a single container or various containers as necessary along with a sheet of instructions for carrying out the test. In some embodiments, the kits also preferably include a positive control sample. Kits can be produced in a variety of ways known in the art.

In one embodiment, the kit comprises a) a set of probes capable of detecting a panel of genes comprising 2, 3, 4, 5, 6, 7, 8, 9, 10, 11, 12, 13, 14, 15, 16, 17, 18, 19, 20, 21, 22, 23, 24, 25, 26, 27, 28, 29, 30, or more genes selected from the group consisting of DEFB4A, S100A7A, SAA2, CCL20, RNASE7, SAA4, IL-23A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2, CSF3, LCN2, SPRR3, DHRS9, GLUL, RHCD, CXCL2, SERPINB3, and S100P; and b) instructions for performing an assay to identify a modulator of the IL-17 pathway. In certain embodiments, the panel of genes consists of DEFB4A, S100A7A, SAA2, CCL20, RNASE7, SAA4, IL-23A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2, and CSF3.

Any of the compositions can be provided in the form of a kit or a reagent mixture. By way of an example, labeled probes can be provided in a kit for the detection of a panel of genes. In certain embodiments, the probes are oligonucleotides. Kits can include all components necessary or sufficient for assays, which can include, but is not limited to, detection reagents (e.g., probes), buffers, control reagents (e.g., positive and negative controls), amplification reagents, solid supports, labels, instruction manuals, etc.

In another aspect, the invention relates to use of a kit of the invention in any method of the invention as claimed.

## EXAMPLES

### Example 1: NHK assay with rhIL-17A stimulation or Th17 conditioned media stimulation

In order to test potential modulators of the IL-17 pathway, normal human keratinocytes (NHK) were incubated with either recombinant IL-17A or Th17 conditioned media and potential inhibitor compounds, and then levels of granulocyte colony-stimulating factor (G-CSF) in the cell supernatant were measured as described in detail below.

### Th17 conditioned media preparation

Peripheral blood mononuclear cells (PBMCs) were isolated from human whole blood using Ficoll-Paque density gradient centrifugation (Ficoll-Paque, GE, 17-1440-03) using SepMate tubes (StemCell technologies, 85450). Then CD4+ T cells were isolated through manual magnetic labeling and cell separation (LS column, Miltenyi Biotec, 130-042-401) following vendor protocol (Miltenyi Biotec, 130-096-533). CD4+ T cells were seeded on 24-well plates at $2x10^6$ cells/mL in 500 $\mu$L of RPMI Media (RPMI 1640, Hyclone, SH30096.01) supplemented with 10% FBS, penicillin-streptomycin (Sigma, P0781-100ml), L-glutamine (Sigma, G7513-100ML), and 2-Mercaptoethanol (Gibco, 21985-023). Th17 polarization media was prepared in RPMI media and 250 $\mu$L was added to each well at a final concentration of: anti-CD3/anti-CD28 beads, prepared using T cell activation/expansion kit (Miltenyi Biotec, 130-091-441), at 2x cell number per well, anti-human interleukin-4 (IL-4) [10 ug/mL], anti-human interferon gamma (IFNγ) [10 ug/mL] antibodies (Thermo-Fisher, 16-7048-85, 16-7318-85), human IL-2 [60 U/mL] (Pepro Tech, 200-02), human IL-1β [10 ng/mL], human IL-23 [10 ng/mL], human IL-6 [50 ng/mL], and human TGFβ [12 ng/mL] (R&D Systems, 201-LB-010, 1290-IL-010, 206-IL-010, 240-B-010). After four days of incubation at 37°C with 5% $CO_2$, the cell culture media in plates was collected and pooled, and used as Th17 conditioned media in NHK assay. The level of human IL-17A in Th17 conditioned media was determined using DuoSet ELISA kits following the vendor protocol (R&D Systems, DY317-05, DY5194-05, DY1335B-05). Human IL-17A was detected in Th17 conditioned media, while unstimulated, undifferentiated CD4+ T cells had no detectable level of IL-17A in the supernatant (FIG. 1).

NHK Treatment

Normal human keratinocytes (Lonza, Basel, Switzerland) were cultured in flasks in keratinocyte growth medium (KGM-Gold Bullet Kit, Lonza) at 37°C with 5% $CO_2$ in a humidified atmosphere. Cells were seeded at 14,000 cells per well of a 96-well plate cell culture plate for transcriptomic analysis or 3-4,000 cells per well of a 384-well plate for IC50 determination. After seeding, the plate was incubated at 37°C with 5% $CO_2$ for 24 hours.

On day 2, media in each well of the cell plate was removed and replaced with fresh KGM media supplemented with 5% fetal bovine serum (FBS). Next, a working concentration of recombinant human IL-17A (Gibco; Gaithersburg, MD) or Th17 conditioned media containing IL-17A was prepared in KGM with 5% FBS. IL-17A or Th17 conditioned media was pre-incubated with titrated compounds (7 concentrations for IC50 determination in NHK G-CSF assay, and selected concentrations for QuantiGene shown in Table 2) or DMSO for 1 hour at room temperature, then was added to the cell culture plate for a final volume per well of 200 μl for 96-well or 100 μl for 384-well plate. The final concentration for recombinant IL-17A was 5 ng/ml, and for IL-17A in Th17 conditioned media was 3-4 ng/ml, and DMSO was 0.2% in the culture. For unstimulated control, cells were treated with culture medium with 0.2% DMSO only.

Table 2

| **Compound/ mAb** | **Description** | **IC50 (NHK G-CSF Assay)** | **IC50 (Literature)** | **Concentration Tested in QuantiGene** | **Structure** |
|---|---|---|---|---|---|
| IL-17Ai-1[1] | IL-17A inhibitor | 57.2 nM[14] | | 9, 0.9, 0.09 μM | 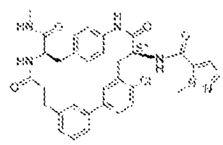 |
| IL-17Ai-2 | IL-17A inhibitor | 55.1 nM[14] | | 5.8, 0.058 μM | 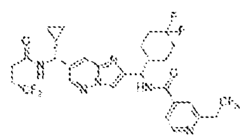 |
| IL-17Ai-3 | IL-17A inhibitor | 72.8 nM[14] | | 1.5, 0.15 μM | 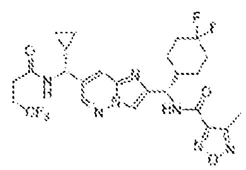 |
| IL-17Ai-4 | IL-17A inhibitor | 57.3 nM[14] | | 5.2, 0.52 μM | 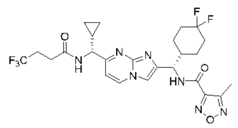 |
| IL-17Ai-5 | IL-17A inhibitor | 86.2 nM[14] | | 7.4, 0.074 μM | 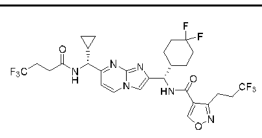 |

(continued)

| Compound/ mAb | Description | IC50 (NHK G-CSF Assay) | IC50 (Literature) | Concentration Tested in QuantiGene | Structure |
|---|---|---|---|---|---|
| IL-17Ai-6 | IL-17A inhibitor | 2.95 nM[14] | | 2.7, 0.027 μM | 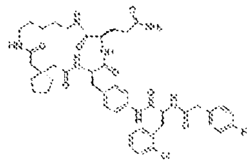 |
| P38i[2] | P38 inhibitor (RWJ 67657) | | 3 nM[4,5], 11 nM[4,6] | 10, 0.7, 0.07 μM | 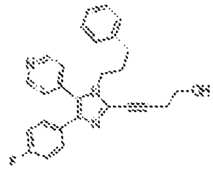 |
| PAKi[3] | PAK inhibitor (PF-3758309) | 2.27 μM[13] | 13.7 nM[7-8], 190 nM[7,9], 99 nM[7,10], 2.7-4.5 nM[7,11], 18.1 nM[7,12], 17.1 nM[7,13] | 20, 1.5, 0.15 μM | 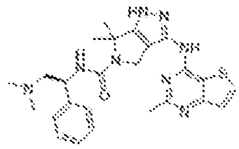 |
| Brodalumab | IL-17 receptor monoclonal antibody | 0.07 nM[14], 0.082 nM[15] | | 7, 0.7, 0.07 nM | |
| B23B90 | Brodalumab isotype control | Inactive | | 7 nM[14], 0.7 nM[15] | |
| Secukinumab | IL-17A monoclonal antibody | 0.2 nM[14-15] | | 20, 2, 0.2 nM | |
| B23B62 | Secukinumab isotype control | Inactive | | 20.6 nM[14], 2 nM[15] | |

1. Liu S, et al., Binding site elucidation and structure guided design of macrocyclic IL-17A antagonists. Sci Rep. 2016 Aug 16;6:30859.
2. p38 mitogen-activated protein kinase inhibitor, Sigma Aldrich, cat#: SML2212-5MG
3. p21-Activated Kinase (PAK) Inhibitor, Sigma Aldrich, cat#: 5006130001
4. Wadsworth SA, Cavender DE, Beers SA, Lalan P, Schafer PH, Malloy EA, et al. RWJ 67657, a potent, orally active inhibitor of p38 mitogen-activated protein kinase. Pharmacol Exp Ther 1999, 291:680-687.
5. Inhibition of TNFa secretion by LPS stimulated hPBMCs
6. Inhibition of IL-1β secretion by LPS stimulated hPBMCs
7. Murray BW, et al. Small-molecule p21-activated kinase inhibitor PF-3758309 is a potent inhibitor of oncogenic signaling and tumor growth. Proc Natl Acad Sci U S A. 2010 May 18;107(20):9446-51
8. Ki of PAK1 kinase domain from enzymatic assay using peptide substrates
9. IC50 of PAK2 kinase domain from enzymatic assay using peptide substrates
10. IC50 of PAK3 kinase domain from enzymatic assay using peptide substrates
11. Kd of PAK4 determined from isothermal calorimetric analysis and surface plasmon resonance
12. Ki of PAK5 kinase domain from enzymatic assay using peptide substrates
13. Ki of PAK5 kinase domain from enzymatic assay using peptide substrates
14. Data from rhIL-17A stimulated NHK assay
15. Data from Th17 conditioned media stimulated NHK

After overnight culture, cell supernatant was carefully collected for G-CSF detection, and cells were further analyzed for viability or gene expression

Detecting G-CSF

G-CSF levels were measured using the Human G-CSF Homogeneous Time Resolved Fluorescence (HTRF) Assay kit (Cisbio, Cat. No. 63ADK055PEBl; Codolet, France) according to the manufacturer's protocol. G-CSF is detected in a sandwich assay format using different specific antibodies, one labeled with Eu3+ - Cryptate (donor) and the second with d2 (acceptor). When the dyes are in close proximity, the excitation of the donor with a light source (laser or flash lamp) triggers a Fluorescence Resonance Energy Transfer (FRET) towards the acceptor, which in turn fluoresces at a specific wavelength (665nm). The two conjugates bind to the antigen present in the sample, thereby generating FRET. Signal intensity is proportional to the number of antigen-antibody complexes formed and therefore to the G-CSF concentration.
Briefly, 16 μl of standard or sample were added per well of a low-volume 384-well HTRF plate (Cisbio, Cat. No. 66PL384100). Then 4 μl of working antibody solution containing anti-G-CSF-d2 conjugate and anti-GCSF-Eu+ cryptate conjugate were added. The plate was incubated overnight at room temperature. Next, the fluorescence emission was read at two different wavelengths (665 nm and 620 nm) using a plate reader. The HTRF ratio was then calculated for each well as [(Signal at 665nm / Signal at 620nm) x 10,000]. Delta F % was then calculated as [((Ratio of standard or sample - Ratio of background)/ Ratio of background)) x 100]. A standard curve was generated for each plate and data was analyzed by using GraphPad Prism version 7.0 Software utilizing a four parameter logistic (4-PL) curve-fit.

Detecting Cell Viability

To test whether treatment with IL-17 or compounds affected cell viability, cells were tested with CellTiter-Glo® Luminescent Cell Viability Assay (Promega; Madison, WI) according to manufacturer's protocol.
The substrate vial and the buffer solution were equilibrated at room temperature before reconstitution. The lyophilized substrate solution was reconstituted by adding the appropriate volume of buffer to the substrate vial. After brief mixing, equal volumes of the reconstituted reagent were added to the volume of media in each well of the plate containing cells. The plate was shaken briefly to mix and incubated at room temperature for 10 minutes then luminescent signal was read using an Envision plate reader.
Percent cell viability was calculated as:

$$\%\ \text{viability} = ((\text{Sample RLU / No stimulation Avg, RLU})*100).$$

As shown in FIG. 2A and 2C, IL-17A stimulated the production of G-CSF in the NHK cells compared to unstimulated cells. While the p38 inhibitor and PAK inhibitor did not inhibit IL-17 stimulated G-CSF production, the IL-17Ai-1 macrocyclic inhibitor, the IL-17 receptor monoclonal antibody, and IL-17 monoclonal antibody inhibited G-CSF production at all concentrations tested (FIG. 2A). Further, small molecule inhibitors IL-17Ai-2 through IL-17Ai-6, all having differing structures, were able to inhibit G-CSF production at all concentrations test (FIG. 2C). Treatment with antibody isotype controls resulted in no inhibition of G-CSF production. As shown in FIG. 2B, IL-17A stimulation did not affect cell viability. The highest dose of 20 μM PAK inhibitor was toxic to the cells, which also had reduced G-CSF production, but other tested concentrations were well tolerated as shown in FIG. 2B. Other compounds did not cause cell toxicity at tested concentrations. These results confirm that the reduction in G-CSF production by IL-17 inhibitors was not caused by cell toxicity.
FIGs. 3A-F show the dose dependent G-CSF levels and corresponding cell viability for p38 inhibitor (FIG 3. A-B), PAK inhibitor (FIG. 3C-D), IL-17Ai-1 (FIG. 3E-F), IL-17Ai-2 (FIGs. 3G-H), IL-17Ai-3 (FIGs. 3I-J), IL-17Ai-4 (FIGs. 3K-L), IL-17Ai-5 (FIGs. 3M-N), and IL-17Ai-6 (FIGs. 3O-P). These results demonstrate that IL-17A inhibitors decrease G-CSF production in a dose-dependent manner while a p38 inhibitor does not inhibit G-CSF levels. PAK inhibitor showed some dose-dependent inhibition of G-CSF production with much lower potency compared to IL-17A inhibitors.
FIG. 4A shows Th17 conditioned media also stimulated G-CSF expression in the media of the NHK cells, while the IL-17 receptor monoclonal antibody and IL-17 monoclonal antibody inhibited G-CSF production. FIG. 4B shows the inhibition of G-CSF production by both antibodies was dose-dependent. These results demonstrate that Th17 conditioned media-stimulated G-CSF production can also be utilized for testing modulators of the IL-17 pathway.

**Example 2: Transcriptomic analysis of IL-17A gene panel**

A panel of IL-17A-stimulated genes was identified, initially through microarray experiments and later confirmed by RT-PCR, to assess modulators of the IL-17 pathway as shown in Table 2. Expression levels of the genes in the panel were measured using QuantiGene® Plex Gene Expression assay in human keratinocytes treated with human recombinant IL-17A and compounds as described in Example 1. Hybridization probes were designed for the 37 genes shown in Table 2.

Table 2

| Full Name | Gene Name | Class |
|---|---|---|
| Homo sapiens chromosome 15 open reading frame 48 | C15orf48 | Target |
| Homo sapiens C-C motif chemokine ligand 20 | CCL20 | Target |
| Homo sapiens colony stimulating factor 3 | CSF3 | Target |
| Homo sapiens C-X-C motif chemokine ligand 1 | CXCL1 | Target |
| Homo sapiens C-X-C motif chemokine ligand 2 | CXCL2 | Target |
| Homo sapiens C-X-C motif chemokine ligand 5 | CXCL5 | Target |
| Homo sapiens beta-defensin 103 | DEFB103A | Target |
| Homo sapiens defensin beta 4A | DEFB4A | Target |
| Homo sapiens Dehydrogenase/reductase SDR family member 9 | DHRS9 | Target |
| Homo sapiens Glutamine synthetase | GLUL | Target |
| Homo sapiens interleukin 23 subunit alpha | IL23A | Target |
| Homo sapiens interleukin 36 gamma | IL36G | Target |
| Homo sapiens Interleukin-8 | IL8 | Target |
| Homo sapiens keratin 78 | KRT78 | Target |
| Homo sapiens lipocalin 2 | LCN2 | Target |
| Homo sapiens NFKB inhibitor zeta | NFKBIZ | Target |
| Homo sapiens PDZK1 interacting protein 1 | PDZK1IP1 | Target |
| Homo sapiens plasminogen activator, tissue type | PLAT | Target |
| Homo sapiens serine protease 22 | PRSS22 | Target |
| Homo sapiens Ammonium transporter Rh type C | RHCG | Target |
| Homo sapiens ribonuclease A family member 7 | RNASE7 | Target |
| Homo sapiens Radical S-adenosyl methionine domain-containing protein 2 | RSAD2 | Target |
| Homo sapiens S100 calcium binding protein A12 | S100A12 | Target |
| Homo sapiens S100 calcium binding protein A7A | S100A7A | Target |
| Homo sapiens Protein S100-P | S100P | Target |
| Homo sapiens serum amvloid A1 | SAA1 | Target |
| Homo sapiens serum amyloid A2 | SAA2 | Target |
| Homo sapiens serum amyloid A4 | SAA4 | Target |
| Homo sapiens serpin family B member 3 | SERPINB3 | Target |
| Homo sapiens Zinc transporter ZIP2 | SLC39A2 | Target |
| Homo sapiens small proline rich protein 2B | SPRR2B | Target |
| Homo sapiens small proline rich protein 3 | SPRR3 | Target |
| Homo sapiens zinc finger CCCH-type containing 12A | ZC3H12A | Target |
| Homo sapiens Actin, cytoplasmic 1 | ACTB | Housekeeping |
| Homo sapiens Glyceraldehyde-3-phosphate dehydrogenase | GAPDH | Housekeeping |
| Homo sapiens Peptidyl-prolyl cis-trans isomerase B | PPIB | Housekeeping |
| Homo sapiens Hypoxanthine-guanine phosphoribosyltransferase | HPRT1 | Housekeeping |

After the 24 hour incubation with IL-17A with and without compounds, 50 μl Lysis buffer (QuantiGene, Thermo-Fisher) was added to each well containing 100 μl cells. The plate was covered and incubated for 30 minutes in a VorTemp

set at 50-55°C, 170 rpm to lyse the cells. The plate was then kept frozen at -80°C. On the day of QuantiGene experiments, samples and reagents were prepared and assay was conducted according the manufacturer's protocol (QuantiGene, ThermoFisher). At the final step, the sample plate was read on a Luminex MagPix® instrument (Luminex; Austin, TX) for fluorescent intensity signals.

The raw expression value of a given gene (g) in a sample (S) was quantified as the median of raw fluorescent intensity of the corresponding probe. Normalization factor of each sample S was determined by the raw expression of four housekeeping genes, ACTB, GAPDH, HPRT1, and PPIB, as the ratio of the geometric mean (geomean) of the four housekeeping genes in sample S vs. the geomean of the four housekeeping genes among all samples. The normalized expression value of each gene in sample S was further quantified as the raw expression value divided by the normalization factor in sample S.

Changes in expression of gene g in keratinocytes in response to IL-17A stimulation was calculated as the ratio of the geomean of its normalized expression values in IL-17A stimulated samples vs. that in unstimulated samples. For each gene g, the relative expression value in sample S (Rel_Exp_S) was further determined by its normalized expression in sample S (Norm_Exp_S) and the geomeans of its normalized expression in IL-17A stimulated samples (GM_Norm_Exp_Stim) and unstimulated samples (GM_Norm_Exp_UnStim), using formula as: Rel_Exp_S = (Norm_Exp_S - GM_Norm_Exp_UnStim) / (GM_Norm_Exp_Stim - GM_Norm_Exp_UnStim). For a given compound or antibody, the % inhibition of induction in gene expression is calculated as (1 - average of relative expression) x 100, among all IL-17A induced genes (with fold change > 1.5).

FIGs. 5A and 5B show heat maps of gene expression levels for the panel of genes in NHKs stimulated with recombinant IL-17A. Small molecule modulators IL-17Ai-1 through IL-17Ai-6, Brodalumab, and Secukinumab all inhibited expression of the gene panel in a dose-dependent manner, demonstrating that this gene panel can be used to identify modulators of the IL-17 or IL-17 receptor. Further, the isotype controls for Brodalumab and Secukinumab show no inhibition of gene expression, confirming the changes are specific to Brodalumab or Secukinumab treatment. Treatment of the p38 and PAK inhibitors did not lead to an overall reduction in expression of the gene panel, instead, they showed mixed effect of augmenting some genes and inhibiting others, demonstrating that this IL-17 gene panel can distinguish inhibitors of targets downstream of IL-17 activation from those upstream like Secukinumab (anti-IL-17A) and Brodalumab (anti-IL-17 receptor), and can be used to identify the IL-17 pathway-specific modulators.

FIG. 6 shows a heatmap of gene expression levels for the panel of genes in NHKs stimulated with Th17 conditioned media. The heatmap demonstrates that Th17 conditioned media stimulates the panel of genes and Brodalumab and Secukinumab inhibited expression of the gene panel in a dose-dependent manner. Further, the isotype controls for Brodalumab and Secukinumab show no inhibition of gene expression, confirming the changes are specific to Brodalumab or Secukinumab treatment.

## Claims

1. A method of identifying an agent that modulates the interleukin-17 (IL-17) pathway, the method comprising:

    a) contacting a cell with IL-17, preferably IL-17A, or T cell conditioned media;
    b) contacting the cell with the agent;
    c) incubating the cell with the IL-17 or T cell conditioned media and the agent;
    d) collecting the cell after the incubating step;
    e) detecting an expression level of a panel of genes of the cell consisting of DEFB4A, S100A7A, SAA2, CCL20, RNASE7, SAA4, IL-23A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2, and CSF3, after the incubating step;
    f) comparing the expression level of the panel of genes of the cell contacted with the agent with a control cell, wherein a modulation in the expression level of the one or more gene(s) in the cell contacted with the agent as compared to the control cell verifies that the agent is capable of modulating the IL-17 pathway.

2. The method of claim 1, further comprising:

    g) collecting the cell supernatant after the incubating step;
    h) detecting a level of granulocyte colony-stimulating factor (G-CSF) in the cell supernatant;
    i) comparing the level of G-C SF in the cell supernatant contacted with the agent with a control cell supernatant, wherein a modulation in the level of G-CSF in the cell supernatant contacted with the agent as compared to the control cell supernatant indicates that the agent is capable of modulating the IL-17 pathway.

3. The method of claim 2, wherein:

(a) the cell supernatant is collected 24 hours after contacting the cell with the agent; and/or
(b) a level of G-CSF is measured using an antibody; and/or
(c) a level of G-CSF is measured using a homogeneous time-resolved fluorescence assay; and/or
(d) the agent decreases the level of G-CSF in the cell supernatant.

4. The method of claim 1, wherein the cell is incubated with the IL-17 or T cell conditioned media and the agent for 6 to 72 hours.

5. The method of any one of claims 1-4, wherein:

(a) the method comprises contacting the cell with IL-17 and the agent sequentially; or
(b) the method comprises contacting the cell with IL-17 and the agent concurrently.

6. The method of any one of claims 1-5, wherein:

(a) the IL-17 is recombinant IL-17; preferably recombinant human IL-17A; or
(b) the T cell conditioned media is T helper 17 (Th17) cell conditioned media.

7. The method of any one of claims 1-6, wherein the method comprises contacting the cell with 0.2 ng/ml to 200 ng/ml IL-17; preferably, the method comprises contacting the cell with 5 ng/ml IL-17.

8. The method of any one of claims 1-7, wherein the agent is selected from the group consisting of a small molecule, a polypeptide, an antibody, and a polynucleotide; optionally wherein the agent has an IC50 of 1 μM or less.

9. The method of any one of claims 1-7, wherein the agent is a small molecule.

10. The method of any one of claims 1-7, wherein the agent is an antibody.

11. The method of claim 9, wherein the agent is an IL-17Ai-1 to 6 selected from the group consisting of the following structures and pharmaceutically acceptable salts thereof:

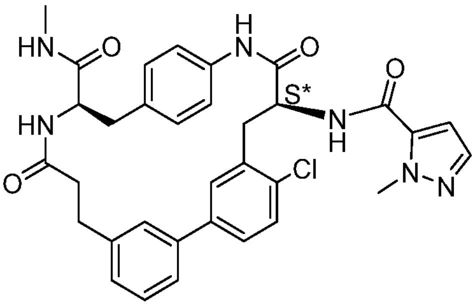

(IL-17Ai-1)

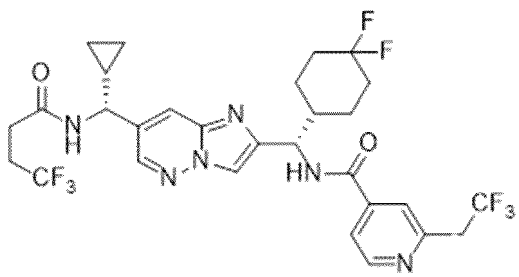

(IL-17Ai-2)

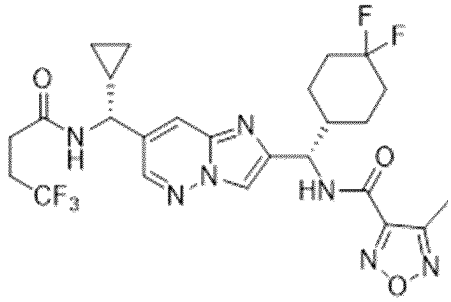

(IL-17Ai-3)

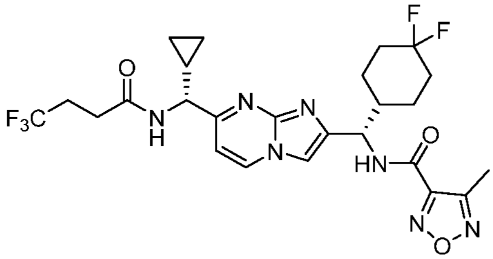

(IL-17Ai-4)

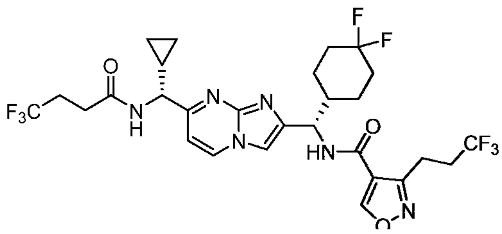

(IL-17Ai-5)

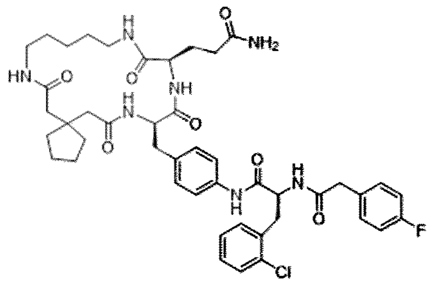

(IL-17Ai-6).

**12.** The method of any one of claims 1-11, wherein:

(a) the cell is incubated with the IL-17 and the agent for 18 to 24 hours in the incubating step; and/or
(b) the cell is a human cell; and/or
(c) the cell is a keratinocyte or a fibroblast cell, preferably a human keratinocyte, optionally wherein the method further comprises growing the human keratinocyte in a keratinocyte growth medium until reaching about 70-90% confluence prior to contacting the cell with the IL-17.

**13.** A kit for identifying a modulator of the interleukin-17 (IL-17) pathway, the kit comprising:

a) only one set of probes capable of detecting only a panel of genes consisting of DEFB4A, S100A7A, SAA2,

CCL20, RNASE7, SAA4, IL-23A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2, and CSF3; and
b) instructions for performing an assay to identify a modulator of the IL-17 pathway.

**14.** The kit of claim 13, wherein the probes are oligonucleotides.

**15.** Use of the kit of claim 13 or 14 in the method of any one of claims 1-13.

## Patentansprüche

**1.** Verfahren zum Identifizieren eines Mittels, das den Interleukin-17-Signalweg (IL-17-Signalweg) moduliert, wobei das Verfahren umfasst:

a) Inkontaktbringen einer Zelle mit IL-17, vorzugsweise IL-17A, oder T-Zell-konditioniertem Medium;
b) Inkontaktbringen der Zelle mit dem Mittel;
c) Inkubieren der Zelle mit dem IL-17 oder T-Zell-konditioniertem Medium und dem Mittel;
d) Sammeln der Zelle nach dem Inkubationsschritt;
e) Nachweisen eines Expressionsspiegels eines Panels von Genen der Zelle bestehend aus DEFB4A, S100A7A, SAA2, CCL20, RNASE7, SAA4, IL-23 A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2 und CSF3 nach dem Inkubationsschritt;
f) Vergleichen des Expressionsspiegels des Panels von Genen der mit dem Mittel in Kontakt gebrachten Zelle mit einer Kontrollzelle, wobei eine Modulation des Expressionsspiegels des einen oder der mehreren Gene in der mit dem Mittel in Kontakt gebrachten Zelle im Vergleich zu der Kontrollzelle verifiziert, dass das Mittel fähig ist, den IL-17-Signalweg zu modulieren.

**2.** Verfahren nach Anspruch 1, ferner umfassend:

g) Sammeln des Zellüberstands nach dem Inkubationsschritt;
h) Nachweisen eines Spiegels von Granulozytenkolonie-stimulierendem Faktor (G-CSF) in dem Zellüberstand;
i) Vergleichen des G-CSF-Spiegels in dem mit dem Mittel in Kontakt gebrachten Zellüberstand mit einem Kontrollzellüberstand, wobei eine Modulation des G-CSF-Spiegels in dem mit dem Mittel in Kontakt gebrachten Zellüberstand im Vergleich zu dem Kontrollzellüberstand angibt, dass das Mittel fähig ist, den IL-17-Signalweg zu modulieren.

**3.** Verfahren nach Anspruch 2, wobei:

(a) der Zellüberstand 24 Stunden nach dem Inkontaktbringen der Zelle mit dem Mittel gesammelt wird; und/oder
(b) ein G-CSF-Spiegel unter Verwendung eines Antikörpers gemessen wird; und/oder
(c) ein G-CSF-Spiegel unter Verwendung eines homogenen zeitaufgelösten Fluoreszenz-Assays gemessen wird; und/oder
(d) das Mittel den G-CSF-Spiegel in dem Zellüberstand verringert.

**4.** Verfahren nach Anspruch 1, wobei die Zelle mit dem IL-17 oder T-Zell-konditioniertem Medium und dem Mittel für 6 bis 72 Stunden inkubiert wird.

**5.** Verfahren nach einem der Ansprüche 1 bis 4, wobei:

(a) das Verfahren das sequenzielle Inkontaktbringen der Zelle mit IL-17 und dem Mittel umfasst; oder
(b) das Verfahren das gleichzeitige Inkontaktbringen der Zelle mit IL-17 und dem Mittel umfasst.

**6.** Verfahren nach einem der Ansprüche 1 bis 5, wobei:

(a) das IL-17 rekombinantes IL-17 ist; vorzugsweise rekombinantes humanes IL-17A; oder
(b) das T-Zell-konditionierte Medium ein T-Helfer-17-(Th17)-Zellkonditioniertes Medium ist.

**7.** Verfahren nach einem der Ansprüche 1 bis 6, wobei das Verfahren das Inkontaktbringen der Zelle mit 0,2 ng/ml bis

200 ng/ml IL-17 umfasst; vorzugsweise das Verfahren das Inkontaktbringen der Zelle mit 5 ng/ml IL-17 umfasst.

**8.** Verfahren nach einem der Ansprüche 1 bis 7, wobei das Mittel ausgewählt ist aus der Gruppe bestehend aus einem kleinen Molekül, einem Polypeptid, einem Antikörper und einem Polynukleotid; wobei optional das Mittel eine IC50 von 1 μM oder weniger aufweist.

**9.** Verfahren nach einem der Ansprüche 1 bis 7, wobei das Mittel ein kleines Molekül ist.

**10.** Verfahren nach einem der Ansprüche 1 bis 7, wobei das Mittel ein Antikörper ist.

**11.** Verfahren nach Anspruch 9, wobei das Mittel ein IL-17Ai-1 bis 6 ist, ausgewählt aus der Gruppe bestehend aus den folgenden Strukturen und pharmazeutisch verträglichen Salzen davon:

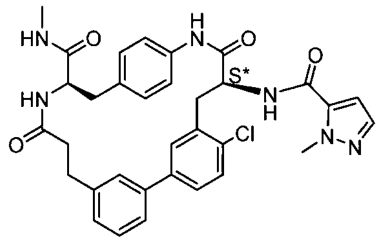

(IL-17 Ai-1)

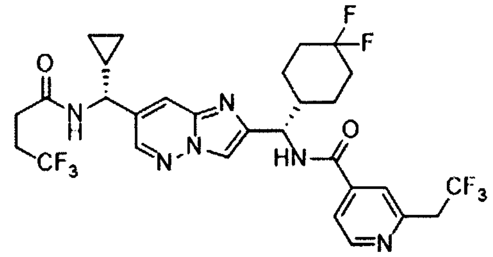

(IL-17 Ai-2)

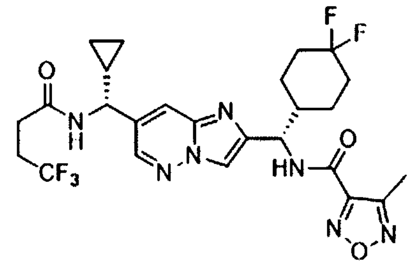

(IL-17 Ai-3)

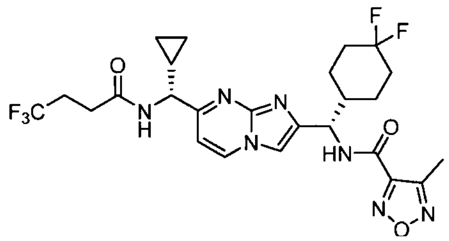

(IL-17 Ai-4)

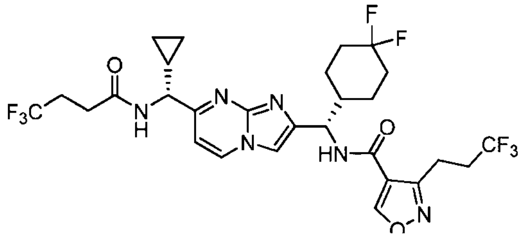

(IL-17 Ai-5)

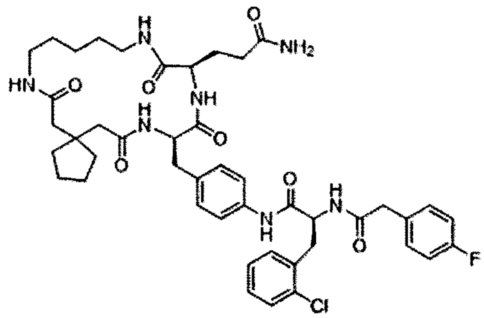

(IL-17 Ai-6).

**12.** Verfahren nach einem der Ansprüche 1 bis 11, wobei:

(a) die Zelle mit dem IL-17 und dem Mittel für 18 bis 24 Stunden in dem Inkubationsschritt inkubiert wird; und/oder
(b) die Zelle eine menschliche Zelle ist; und/oder
(c) die Zelle eine Keratinozyte oder eine Fibroblastenzelle ist, vorzugsweise eine humane Keratinozyte, wobei optional das Verfahren ferner das Züchten der humanen Keratinozyte in einem Keratinozytenwachstumsmedium bis zum Erreichen von etwa 70-90 % Konfluenz vor dem Inkontaktbringen der Zelle mit dem IL-17 umfasst.

**13.** Kit zum Identifizieren eines Modulators des Interleukin-17-Signalwegs (IL-17-Signalwegs), wobei das Kit umfasst:

a) nur einen Satz von Sonden, die fähig sind, nur ein Panel von Genen nachzuweisen, bestehend aus: DEFB4A, S100A7A, SAA2, CCL20, RNASE7, SAA4, IL-23 A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2 und CSF3; und
b) Anweisungen zum Durchführen eines Assays zum Identifizieren eines Modulators des IL-17-Signalwegs.

**14.** Kit nach Anspruch 13, wobei die Sonden Oligonukleotide sind.

**15.** Verwendung des Kits nach Anspruch 13 oder 14 in dem Verfahren nach einem der Ansprüche 1 bis 13.

## Revendications

1. **Procédé** d'identification d'un agent qui module la voie d'interleukine-17 (IL-17), le procédé comprenant :

   a) la mise en contact d'une cellule avec IL-17, de préférence IL-17A, ou un milieu conditionné de cellules T ;
   b) la mise en contact de la cellule avec l'agent ;
   c) l'incubation de la cellule avec l'IL-17 ou du milieu conditionné de cellules T et l'agent ;
   d) la collecte de la cellule après l'étape d'incubation ;
   e) la détection d'un niveau d'expression d'un panel de gènes de la cellule constitué de DEFB4A, S100A7A, SAA2, CCL20, RNASE7, SAA4, IL-23 A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2 et CSF3, après l'étape d'incubation ;
   f) la comparaison du niveau d'expression du panel de gènes de la cellule mise en contact avec l'agent avec une cellule témoin, dans lequel une modulation dans le niveau d'expression du ou des gènes dans la cellule mise en contact avec l'agent par comparaison avec la cellule témoin vérifie que l'agent est capable de moduler la voie d'IL-17.

2. Procédé selon la revendication 1, comprenant en outre :

   g) la collecte du surnageant cellulaire après l'étape d'incubation ;
   h) la détection d'un niveau de facteur de stimulation de colonies de granulocytes (G-CSF) dans le surnageant cellulaire ;
   i) la comparaison du niveau de G-CSF dans le surnageant cellulaire mis en contact avec l'agent à un surnageant cellulaire témoin, dans lequel une modulation dans le niveau de G-CSF dans le surnageant cellulaire mis en contact avec l'agent par comparaison avec le surnageant cellulaire témoin indique que l'agent est capable de moduler la voie d'IL-17.

3. Procédé selon la revendication 2, dans lequel :

   (a) le surnageant cellulaire est collecté 24 heures après mise en contact de la cellule avec l'agent ; et/ou
   (b) un niveau de G-CSF est mesuré à l'aide d'un anticorps ; et/ou
   (c) un niveau de G-CSF est mesuré à l'aide d'un dosage de fluorescence à résolution temporelle homogène ; et/ou
   (d) l'agent diminue le niveau de G-CSF dans le surnageant cellulaire.

4. Procédé selon la revendication 1, dans lequel la cellule est incubée avec l'IL-17 ou le milieu conditionné de cellules T et l'agent pendant 6 à 72 heures.

5. Procédé selon l'une quelconque des revendications 1 à 4, dans lequel :

   (a) le procédé comprend la mise en contact de la cellule avec IL-17 et l'agent séquentiellement ; ou
   (b) le procédé comprend la mise en contact de la cellule avec IL-17 et l'agent simultanément.

6. Procédé selon l'une quelconque des revendications 1 à 5, dans lequel :

   (a) l'IL-17 est de l'IL-17 recombiné ; de préférence de l'IL-17A humain recombiné ; ou
   (b) le milieu conditionné de cellules T est un milieu conditionné de cellules T auxiliaires 17 (Thl7).

7. Procédé selon l'une quelconque des revendications 1 à 6, dans lequel le procédé comprend la mise en contact de la cellule avec 0,2 ng/mL à 200 ng/mL d'IL-17 ; de préférence, le procédé comprend la mise en contact de la cellule avec 5 ng/mL d'IL-17.

8. Procédé selon l'une quelconque des revendications 1 à 7, dans lequel l'agent est choisi dans le groupe constitué d'une petite molécule, d'un polypeptide, d'un anticorps et d'un polynucléotide ; facultativement dans lequel l'agent a une IC50 de 1 μM ou moins.

9. Procédé selon l'une quelconque des revendications 1 à 7, dans lequel l'agent est une petite molécule.

**10.** Procédé selon l'une quelconque des revendications 1 à 7, dans lequel l'agent est un anticorps.

**11.** Procédé selon la revendication 9, dans lequel l'agent est un IL-17Ai-1 à 6 choisi dans le groupe constitué des structures suivantes et de sels pharmaceutiquement acceptables de celles-ci :

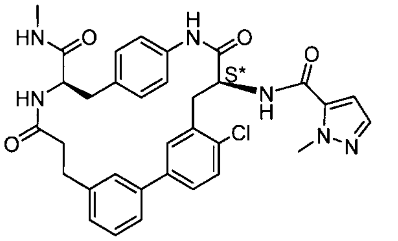

(IL-17 Ai-1)

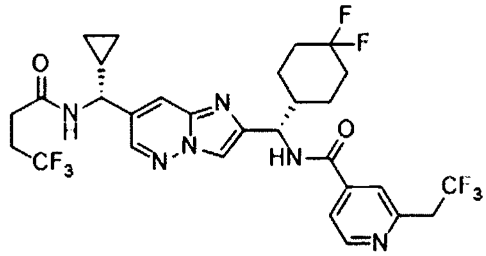

(IL-17 Ai-2)

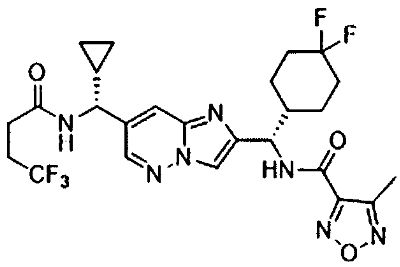

(IL-17Ai-3)

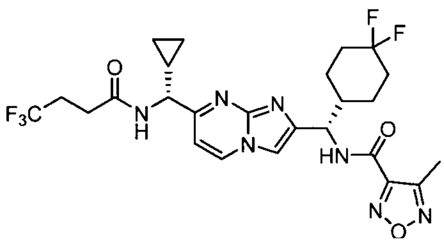

(IL-17 Ai-4)

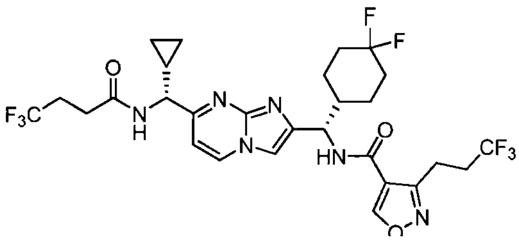

(IL-17Ai-5)

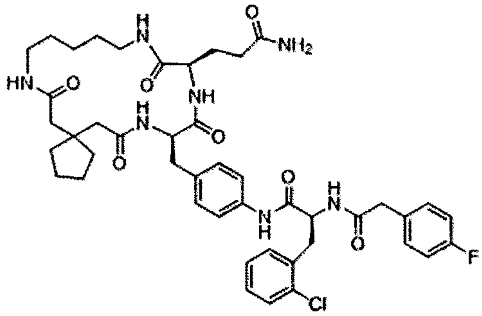

(IL-17Ai-6).

**12.** Procédé selon l'une quelconque des revendications 1 à 11, dans lequel :

(a) la cellule est incubée avec l'IL-17 et l'agent pendant 18 à 24 heures dans l'étape d'incubation ; et/ou
(b) la cellule est une cellule humaine ; et/ou
(c) la cellule est un kératinocyte ou une cellule de fibroblaste, de préférence un kératinocyte humain, facultativement dans lequel le procédé comprend en outre la croissance du kératinocyte humain dans un milieu de croissance de kératinocytes jusqu'à l'obtention d'environ 70 à 90 % de confluence avant la mise en contact de la cellule avec l'IL-17.

**13.** Trousse permettant d'identifier un modulateur de la voie d'interleukine-17 (IL-17), la trousse comprenant :

a) un seul ensemble de sondes capables de ne détecter qu'un panel de gènes constitué de DEFB4A, S100A7A, SAA2, CCL20, RNASE7, SAA4, IL-23 A, S100A12, DEFB103A, C15orf48, ZC3H12A, SAA1, SPRR2B, PRSS22, CXCL1, IL-36G, NFKBIZ, IL-8, PLAT, CXCL5, PDZK1IP1, RSAD2, KRT78, SLC39A2 et CSF3 ; et
b) des instructions permettant de mettre en oeuvre un dosage pour identifier un modulateur de la voie d'IL-17.

**14.** Trousse selon la revendication 13, dans laquelle les sondes sont des oligonucléotides.

**15.** Utilisation de la trousse selon la revendication 13 ou 14 dans le procédé selon l'une quelconque des revendications 1 à 13.

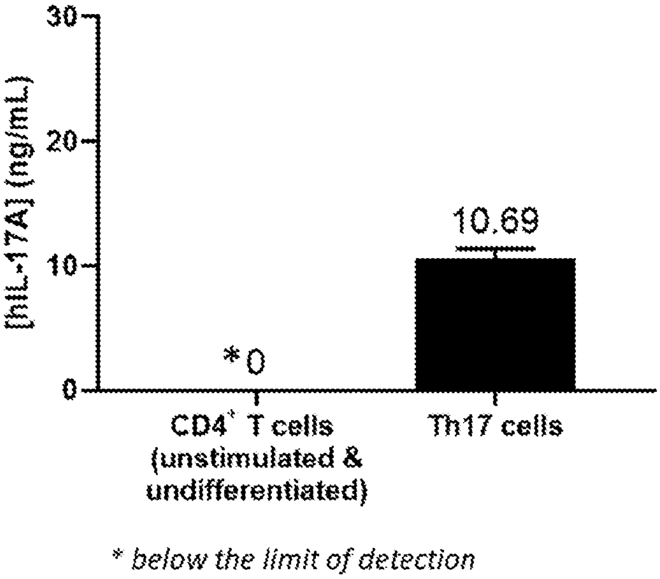
30
20
10
0
[hIL-17A] (ng/mL)
10.69
*0
CD4+ T cells
(unstimulated &
undifferentiated)
Th17 cells
* below the limit of detection

FIG. 1

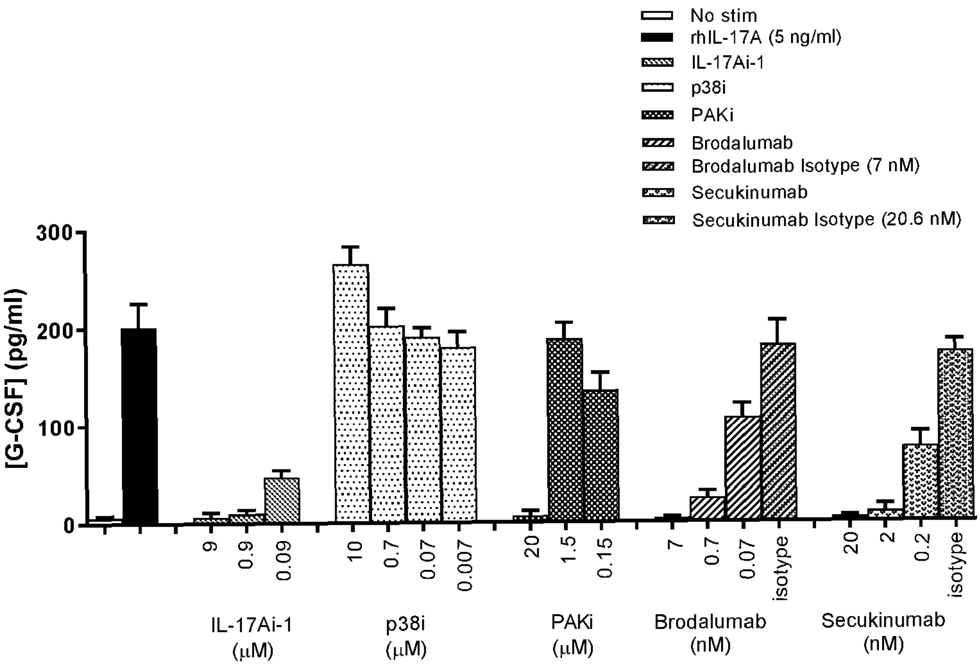
No stim
rhIL-17A (5 ng/ml)
IL-17Ai-1
p38i
PAKi
Brodalumab
Brodalumab Isotype (7 nM)
Secukinumab
Secukinumab Isotype (20.6 nM)
[G-CSF] (pg/ml)
300
200
100
0
9
0.9
0.09
10
0.7
0.07
0.007
20
1.5
0.15
7
0.7
0.07
isotype
20
2
0.2
isotype
IL-17Ai-1 (μM)
p38i (μM)
PAKi (μM)
Brodalumab (nM)
Secukinumab (nM)

FIG. 2A

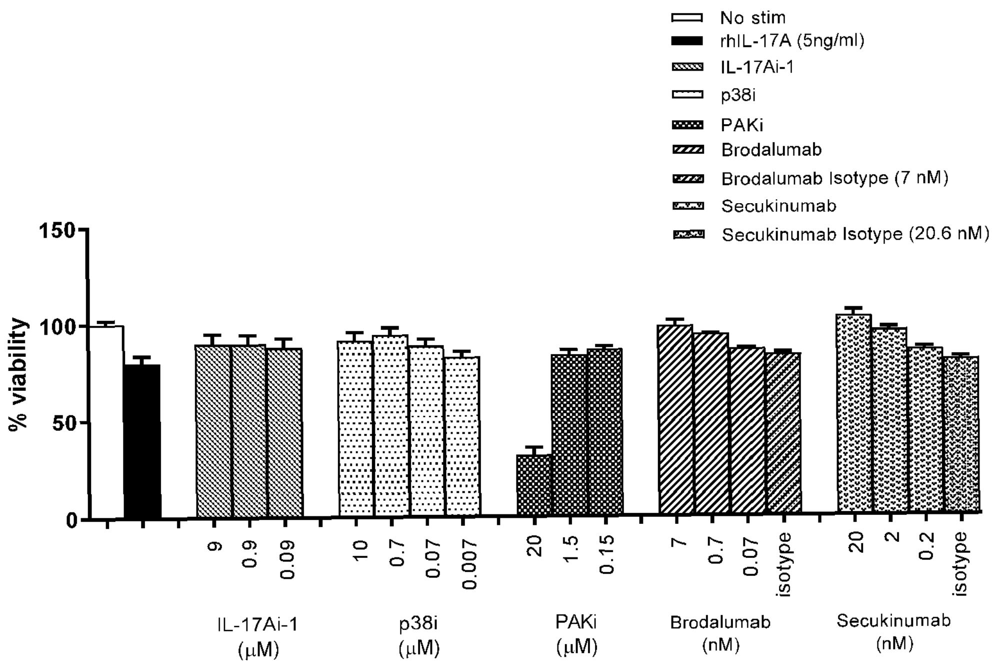
No stim
rhIL-17A (5ng/ml)
IL-17Ai-1
p38i
PAKi
Brodalumab
Brodalumab Isotype (7 nM)
Secukinumab
Secukinumab Isotype (20.6 nM)
% viability
150
100
50
0
9
0.9
0.09
10
0.7
0.07
0.007
20
1.5
0.15
7
0.7
0.07
isotype
20
2
0.2
isotype
IL-17Ai-1 (μM)
p38i (μM)
PAKi (μM)
Brodalumab (nM)
Secukinumab (nM)

FIG. 2B

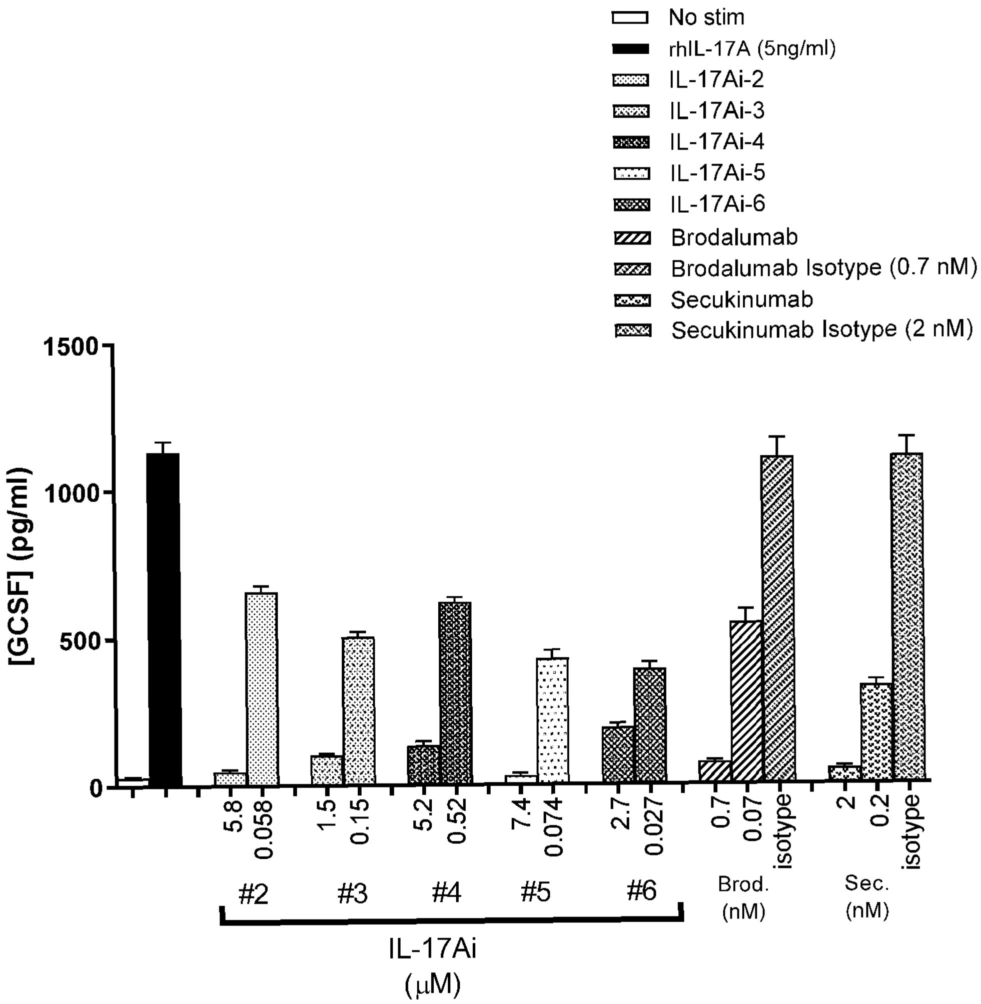
No stim
rhIL-17A (5ng/ml)
IL-17Ai-2
IL-17Ai-3
IL-17Ai-4
IL-17Ai-5
IL-17Ai-6
Brodalumab
Brodalumab Isotype (0.7 nM)
Secukinumab
Secukinumab Isotype (2 nM)
1500
1000
500
0
[GCSF] (pg/ml)
5.8
0.058
1.5
0.15
5.2
0.52
7.4
0.074
2.7
0.027
0.7
0.07
isotype
2
0.2
isotype
#2
#3
#4
#5
#6
Brod.
(nM)
Sec.
(nM)
IL-17Ai
(μM)

FIG. 2C

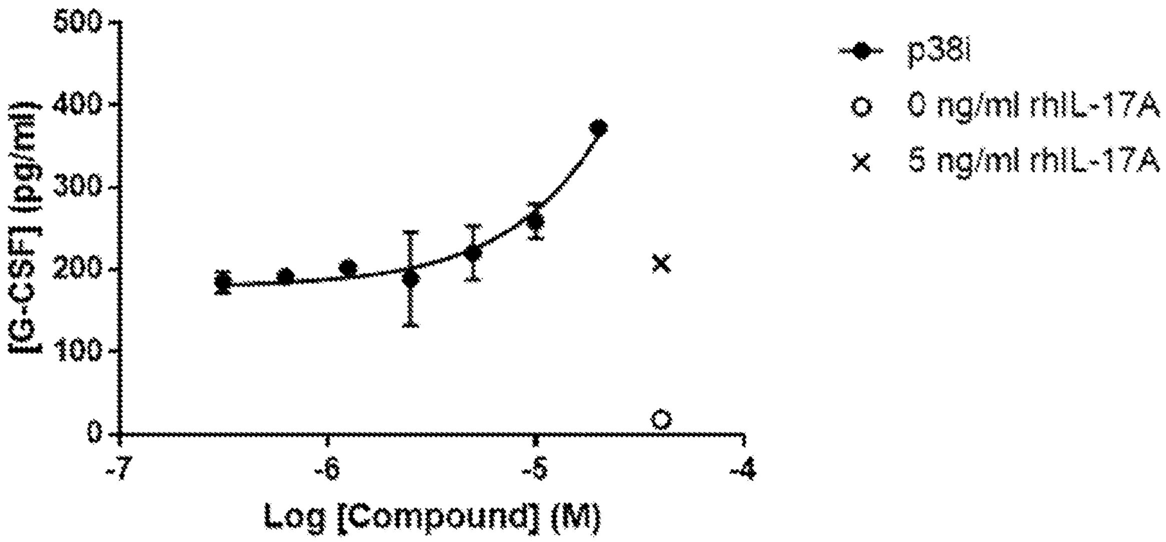
500
400
300
200
100
0
[G-CSF] (pg/ml)
-7
-6
-5
-4
Log [Compound] (M)
p38i
0 ng/ml rhIL-17A
5 ng/ml rhIL-17A

FIG. 3A

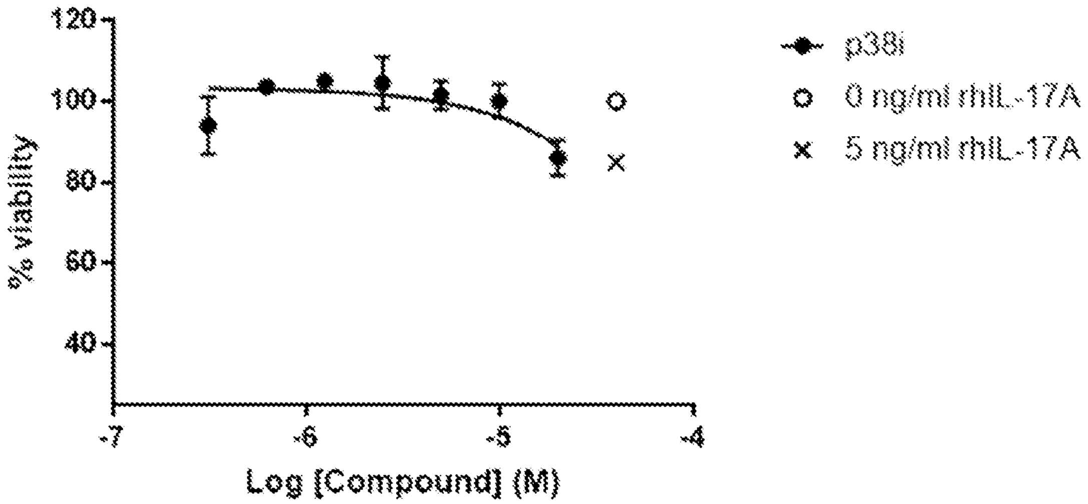
120
100
80
60
40
% viability
-7
-6
-5
-4
Log [Compound] (M)
p38i
0 ng/ml rhIL-17A
5 ng/ml rhIL-17A

FIG. 3B

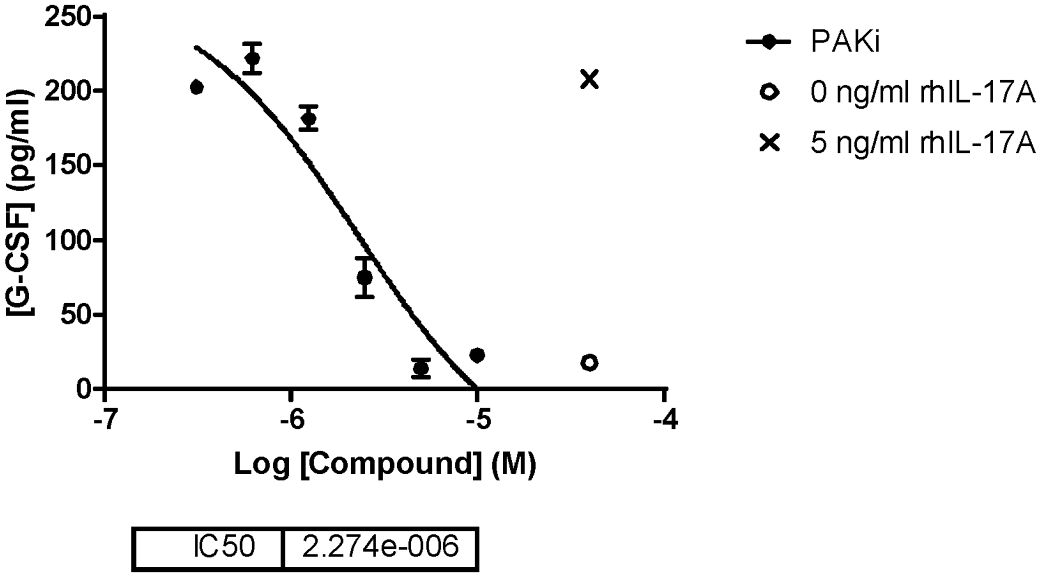

[G-CSF] (pg/ml)
250
200
150
100
50
0
-7
-6
-5
-4
Log [Compound] (M)
PAKi
0 ng/ml rhIL-17A
5 ng/ml rhIL-17A
IC50
2.274e-006


FIG. 3C

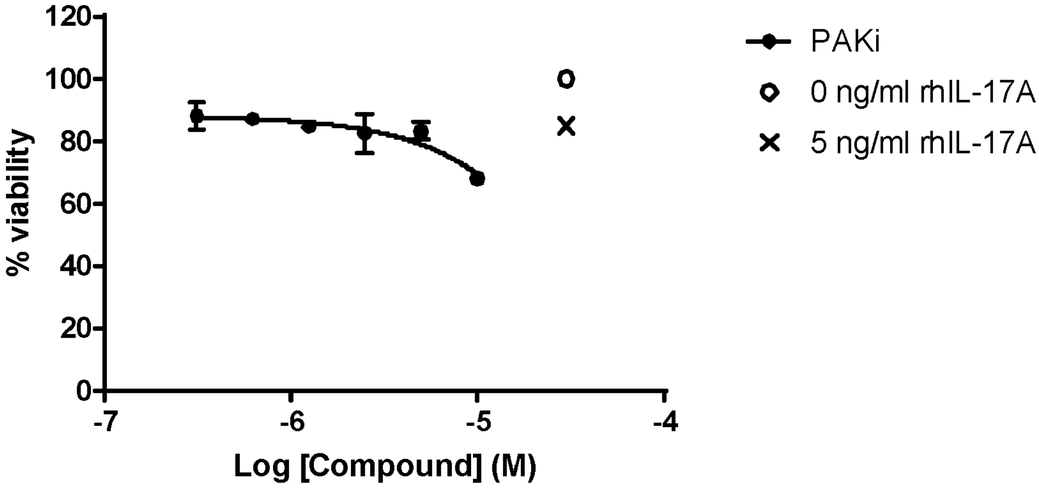

% viability
120
100
80
60
40
20
0
-7
-6
-5
-4
Log [Compound] (M)
PAKi
0 ng/ml rhIL-17A
5 ng/ml rhIL-17A


FIG. 3D

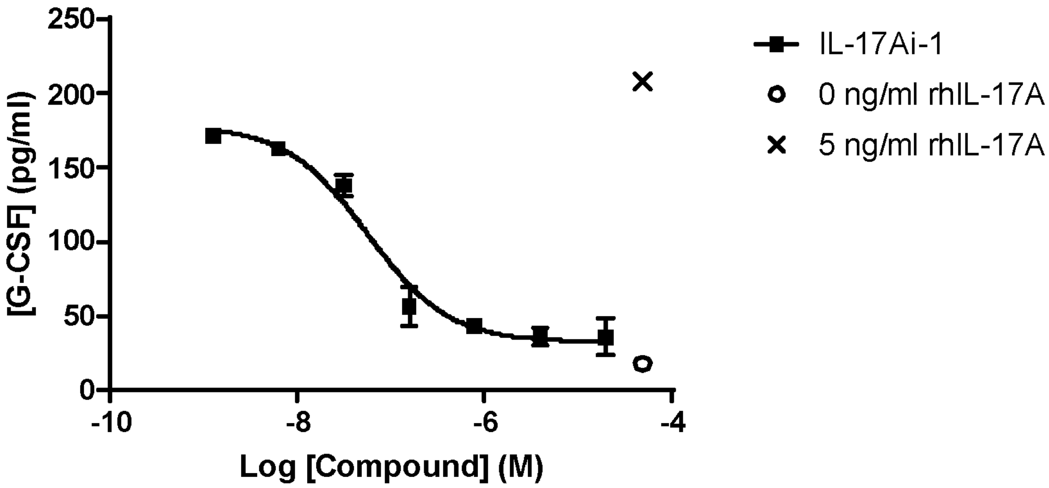


| | IL-17Ai-1 |
|---|---|
| IC50 | 5.722e-008 |

FIG. 3E

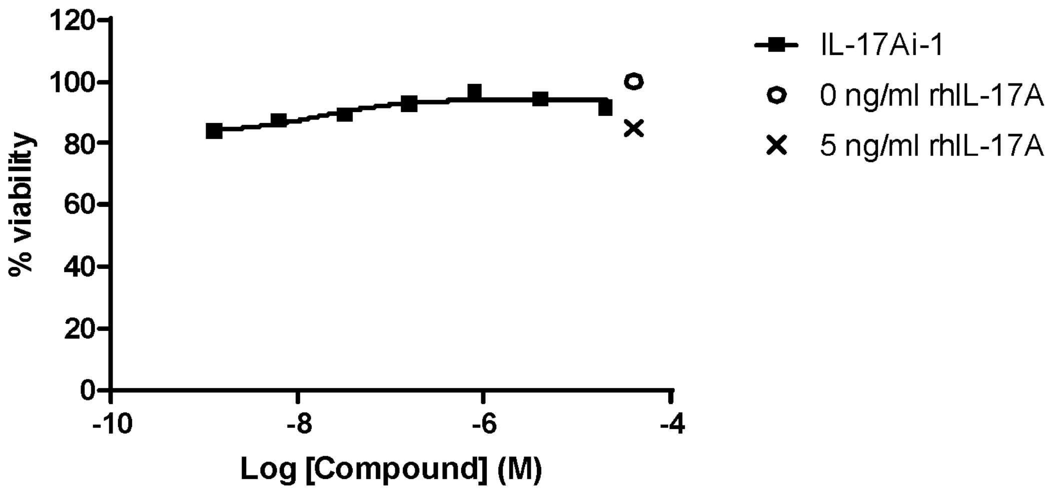


FIG. 3F

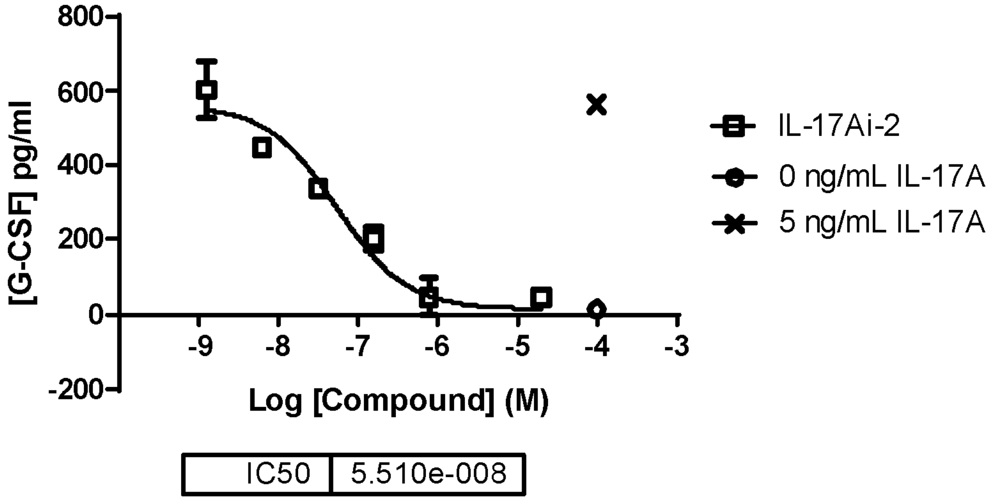
800
600
400
200
0
-200
[G-CSF] pg/ml
-9
-8
-7
-6
-5
-4
-3
Log [Compound] (M)
IL-17Ai-2
0 ng/mL IL-17A
5 ng/mL IL-17A
IC50
5.510e-008

FIG. 3G

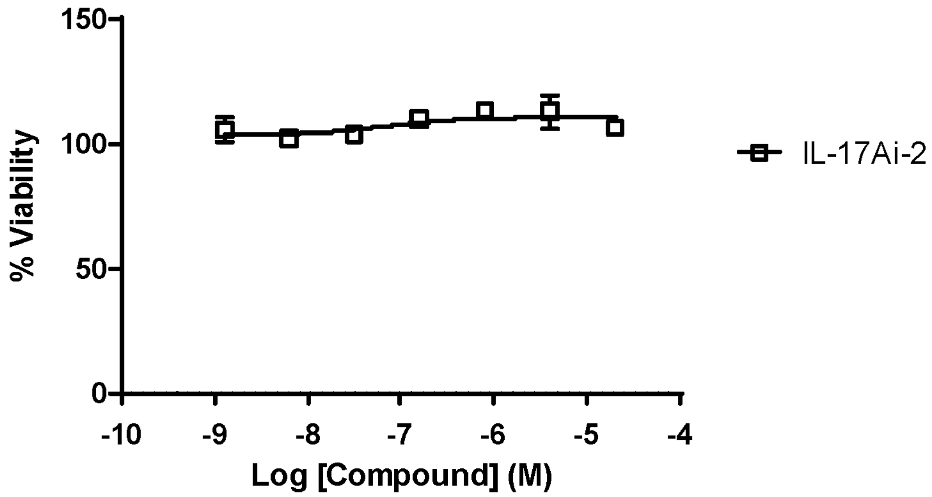
150
100
50
0
% Viability
-10
-9
-8
-7
-6
-5
-4
Log [Compound] (M)
IL-17Ai-2

FIG. 3H

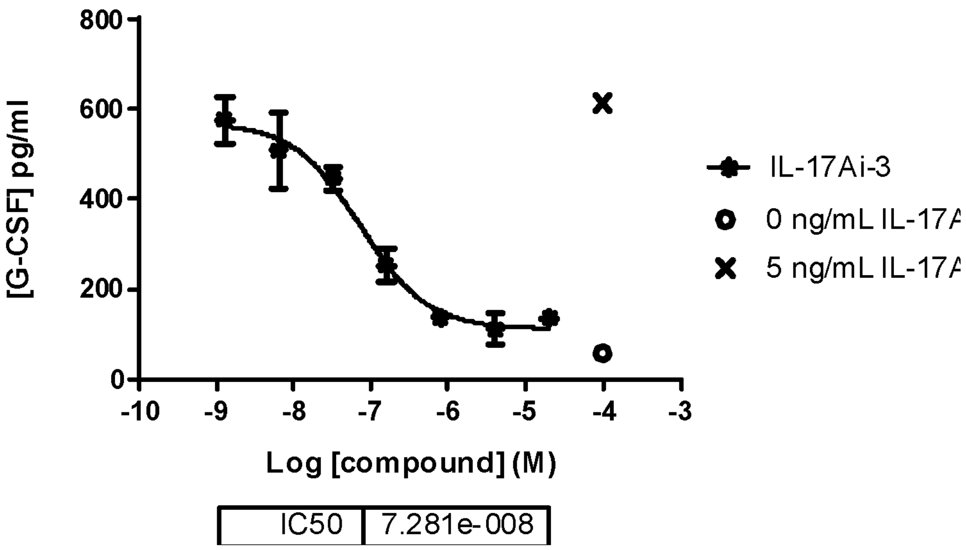
800
600
400
200
0
[G-CSF] pg/ml
-10
-9
-8
-7
-6
-5
-4
-3
Log [compound] (M)
IL-17Ai-3
0 ng/mL IL-17A
5 ng/mL IL-17A
IC50
7.281e-008

FIG. 3I

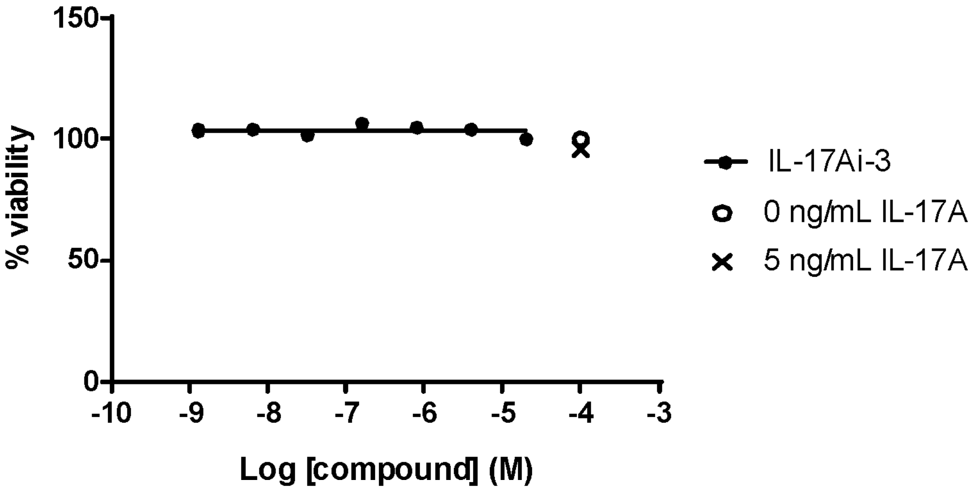
150
100
50
0
% viability
-10
-9
-8
-7
-6
-5
-4
-3
Log [compound] (M)
IL-17Ai-3
0 ng/mL IL-17A
5 ng/mL IL-17A

FIG. 3J

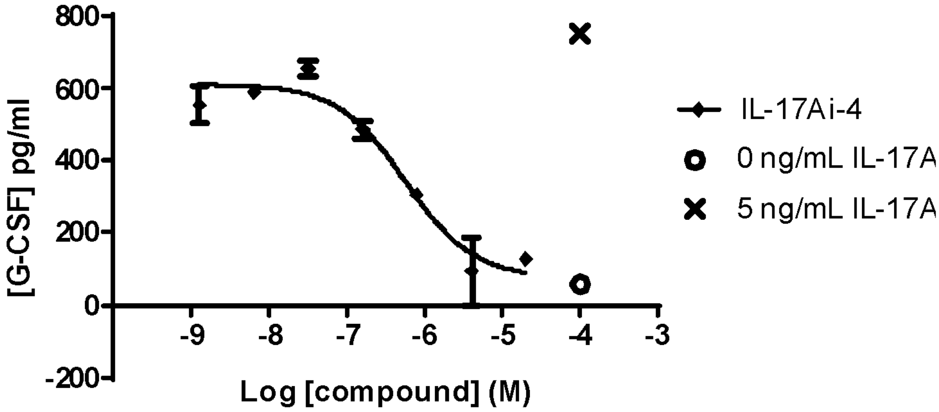


| IC50 | 5.729e-007 |
|---|---|

FIG. 3K

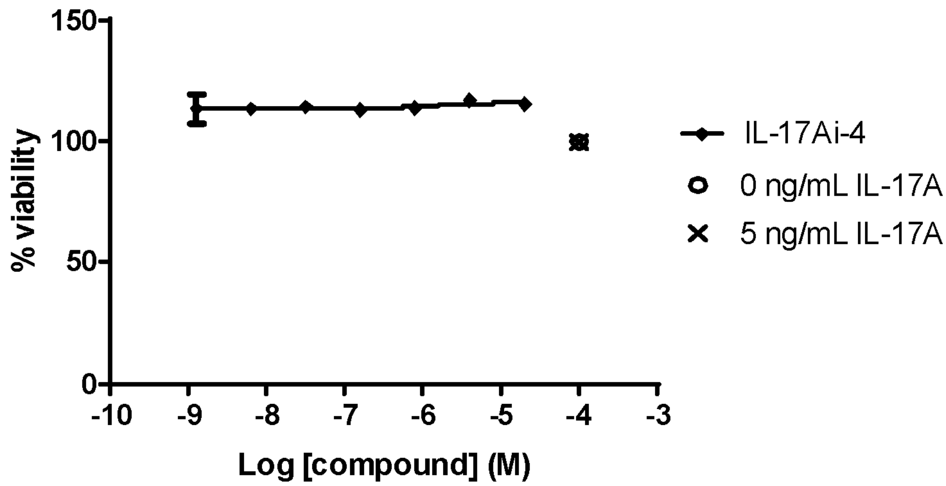


FIG. 3L

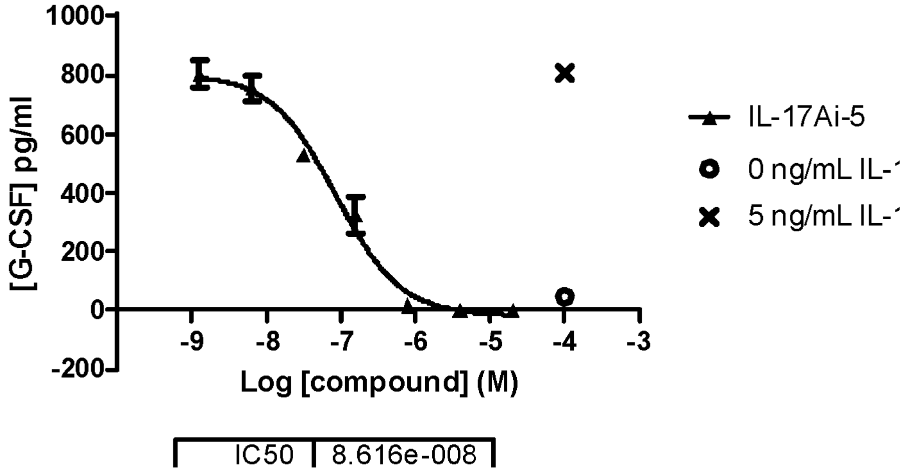
1000
800
600
400
200
0
-200
[G-CSF] pg/ml
-9
-8
-7
-6
-5
-4
-3
Log [compound] (M)
IL-17Ai-5
0 ng/mL IL-
5 ng/mL IL-
IC50
8.616e-008

FIG. 3M

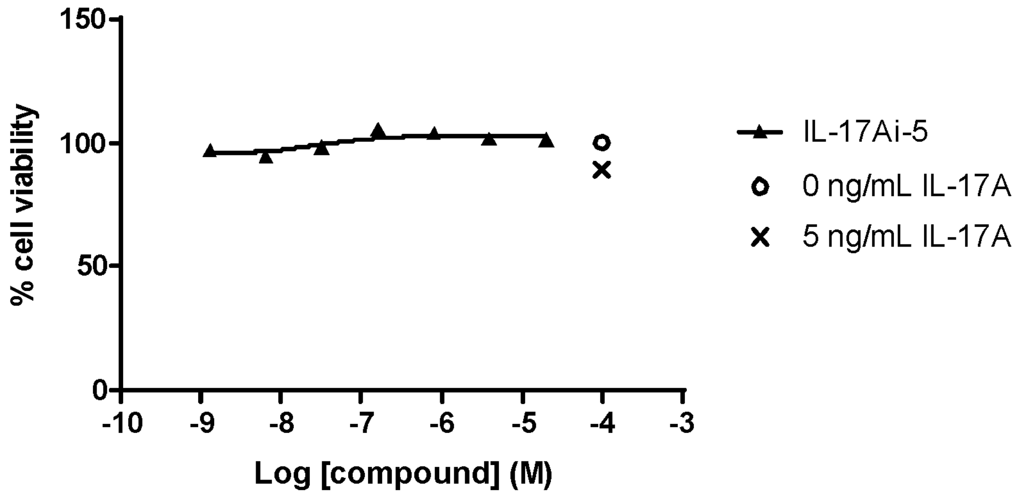
150
100
50
0
% cell viability
-10
-9
-8
-7
-6
-5
-4
-3
Log [compound] (M)
IL-17Ai-5
0 ng/mL IL-17A
5 ng/mL IL-17A

FIG. 3N

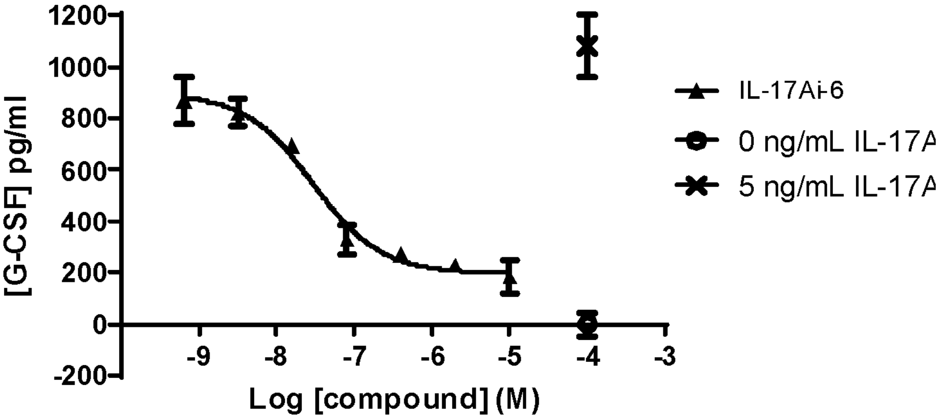


| IC50 | 2.949e-008 |
| --- | --- |

FIG. 3O

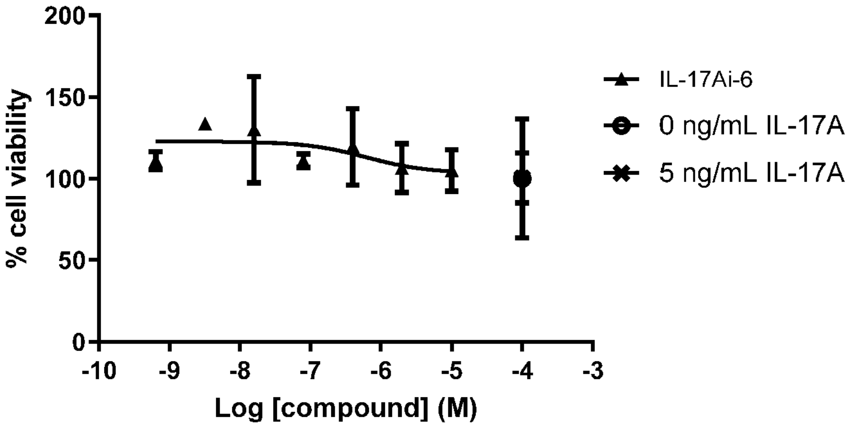


FIG. 3P

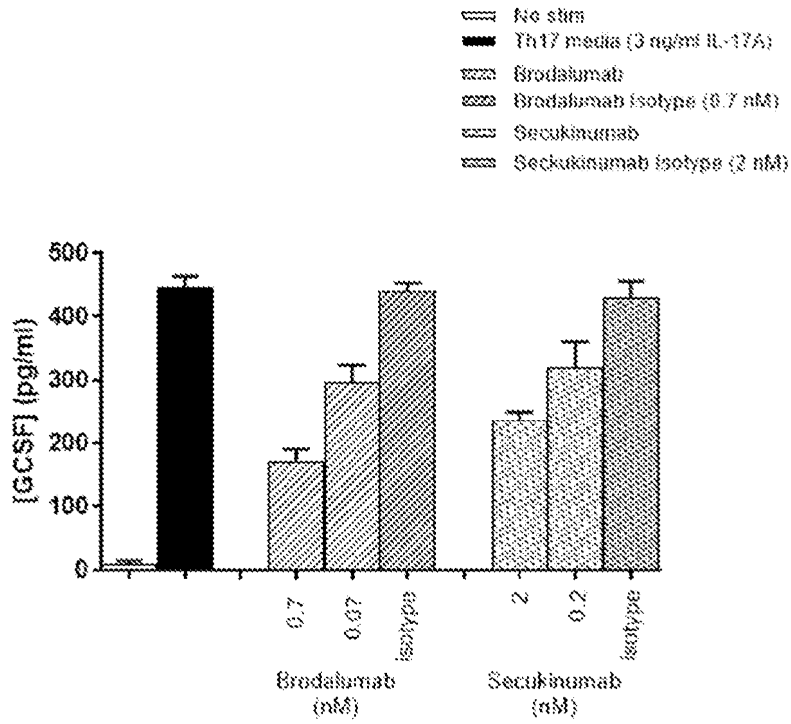


FIG. 4A

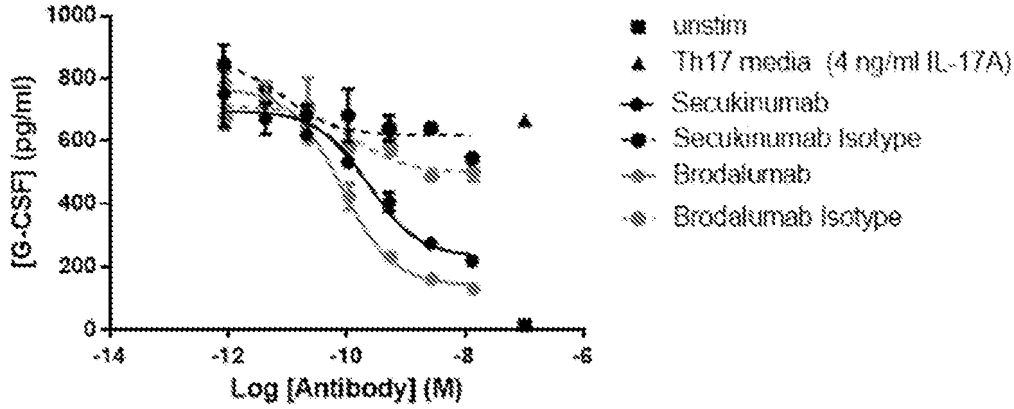


| | Secukinumab | Brodalumab |
|---|---|---|
| IC50 | 2.318e-010 | 8.214e-011 |

FIG. 4B

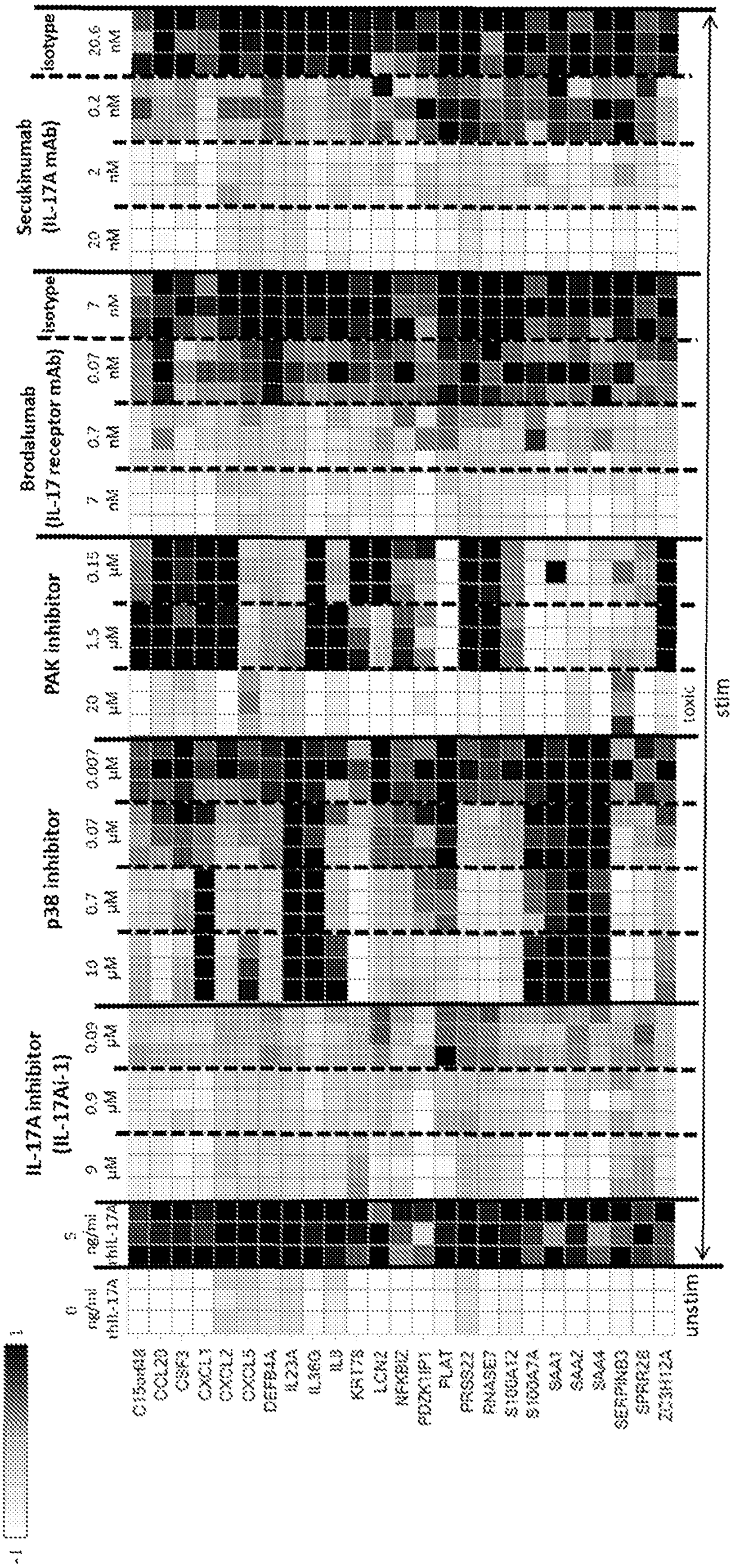

-1
1
0 ng/ml rhIL-17A
5 ng/ml rhIL-17A
IL-17A inhibitor (IL-17Ai-1)
9 µM
0.9 µM
0.09 µM
p38 inhibitor
10 µM
0.7 µM
0.07 µM
0.007 µM
PAK inhibitor
20 µM
1.5 µM
0.15 µM
toxic
Brodalumab (IL-17 receptor mAb)
7 nM
0.7 nM
0.07 nM
isotype
7 nM
Secukinumab (IL-17A mAb)
20 nM
2 nM
0.2 nM
isotype
20.6 nM
C15orf48
CCL20
CSF3
CXCL1
CXCL2
CXCL5
DEFB4A
IL23A
IL36G
IL8
KRT7B
LCN2
NFKBIZ
PDZK1IP1
PLAT
PRSS22
RNASE7
S100A12
S100A7A
SAA1
SAA2
SAA4
SERPINB3
SPRR2B
ZC3H12A
unstim
stim


FIG. 5A

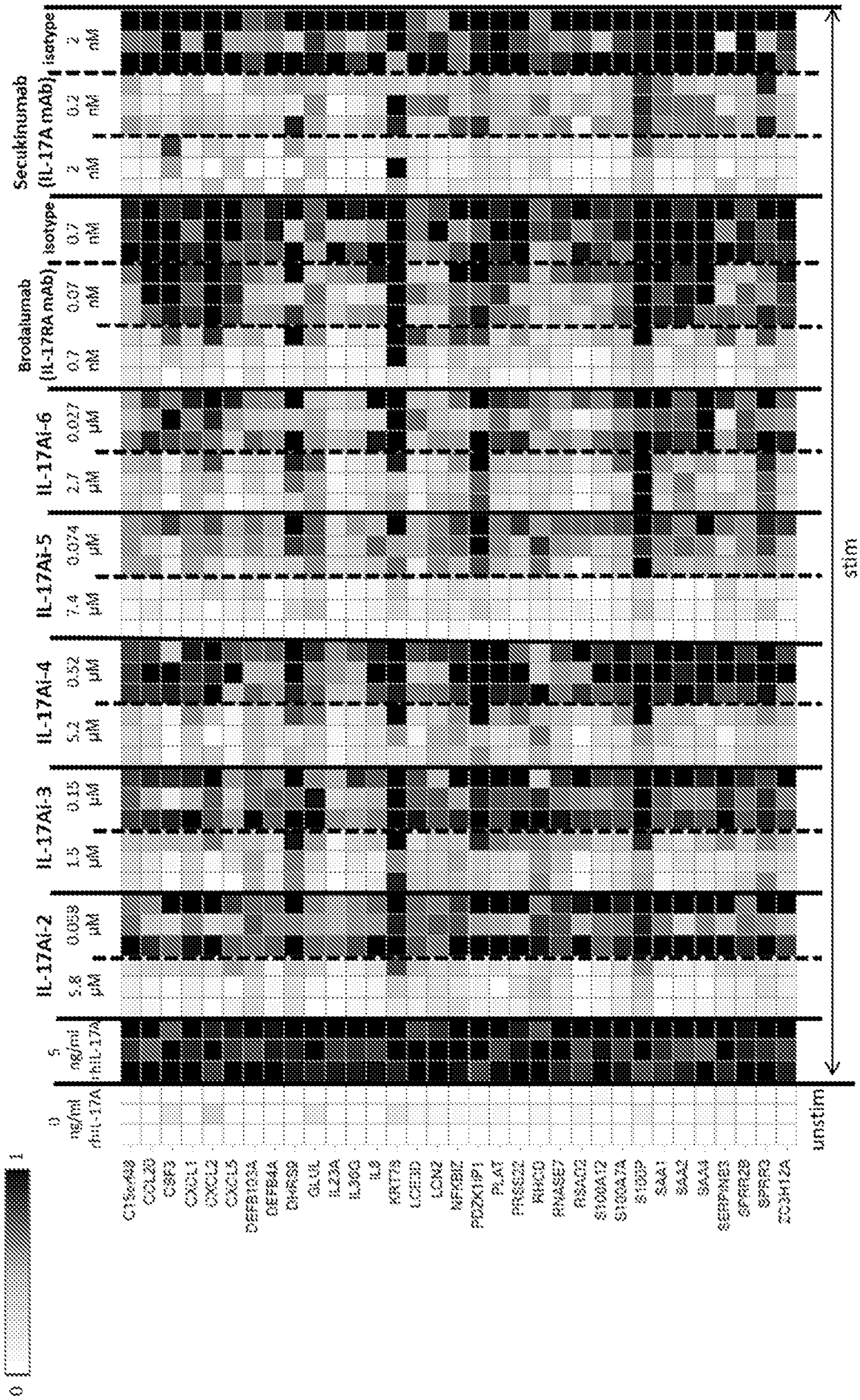
0
1
0 ng/ml rhIL-17A
5 ng/ml rhIL-17A
IL-17Ai-2
5.8 µM
0.058 µM
IL-17Ai-3
1.5 µM
0.15 µM
IL-17Ai-4
5.2 µM
0.52 µM
IL-17Ai-5
7.4 µM
0.074 µM
IL-17Ai-6
2.7 µM
0.027 µM
Brodalumab (IL-17RA mAb)
0.7 nM
0.07 nM
isotype
0.7 nM
Secukinumab (IL-17A mAb)
2 nM
0.2 nM
isotype
2 nM
C15orf48
CCL20
CSF3
CXCL1
CXCL2
CXCL5
DEFB103A
DEFB4A
DHRS9
GLUL
IL23A
IL36G
IL8
KRT78
LCE3D
LCN2
NFKBIZ
PDZK1IP1
PLAT
PRSS22
RHCG
RNASE7
RSAD2
S100A12
S100A7A
S100P
SAA1
SAA2
SAA4
SERPINB3
SPRR2B
SPRR3
ZC3H12A
unstim
stim

FIG. 5B

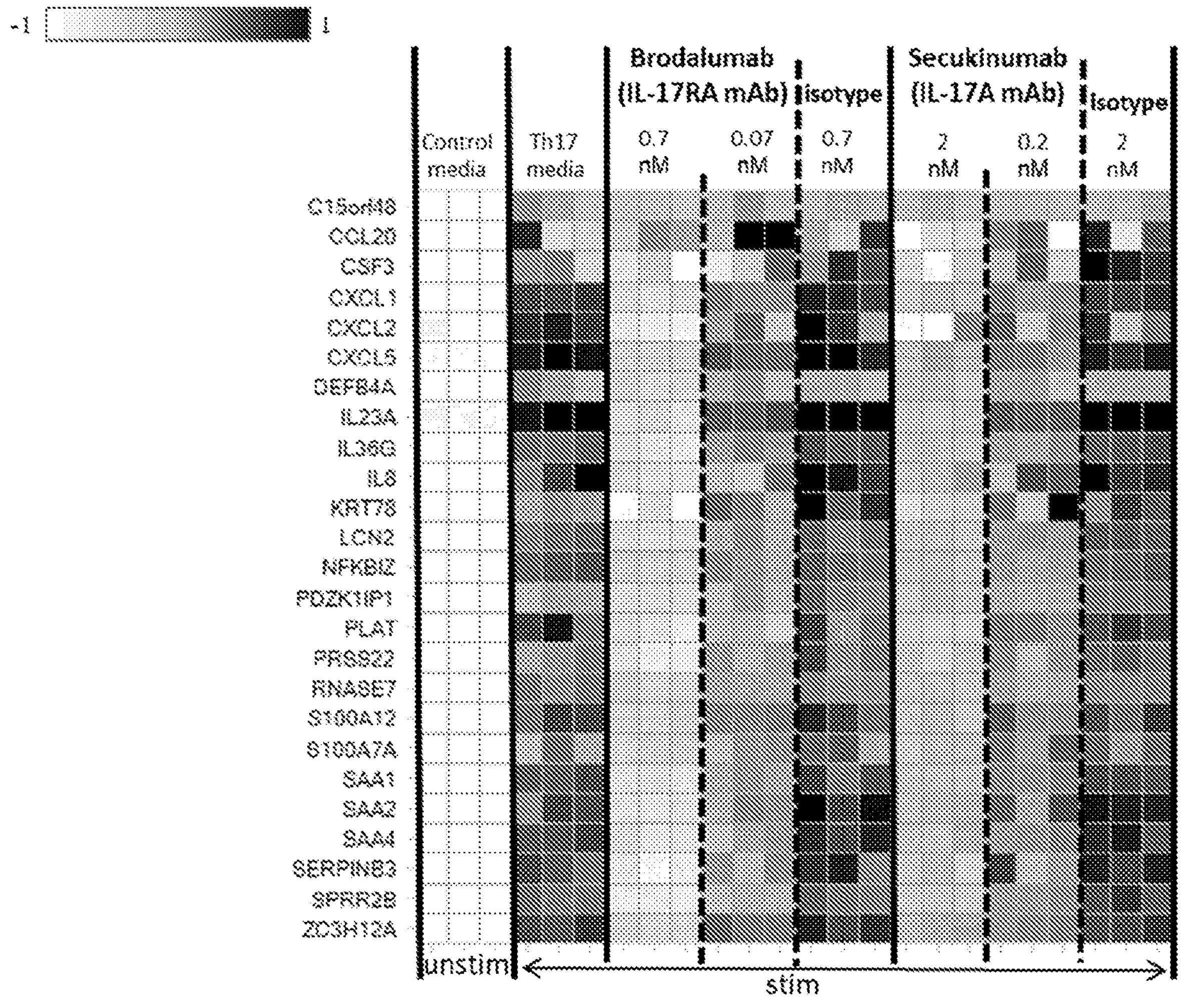

-1
1
Brodalumab (IL-17RA mAb)
isotype
Secukinumab (IL-17A mAb)
isotype
Control media
Th17 media
0.7 nM
0.07 nM
0.7 nM
2 nM
0.2 nM
2 nM
C15orf48
CCL20
CSF3
CXCL1
CXCL2
CXCL5
DEFB4A
IL23A
IL36G
IL8
KRT78
LCN2
NFKBIZ
PDZK1IP1
PLAT
PRSS22
RNASE7
S100A12
S100A7A
SAA1
SAA2
SAA4
SERPINB3
SPRR2B
ZC3H12A
unstim
stim


FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63017960
- US 2016159914 A
- EP 2288382 A2
- US 63017682 B
- US 63017679 B
- WO 2013116682 A


### Non-patent literature cited in the description


- **HARDEN J. et al.** *J Autoimmun*, 2015, vol. 64, 66-73
- **HA H.-L. et al.** *Proc. Natl. Acad. Sci. U.S.A.*, 2014, vol. 111 (33), 3422-3431
- *Pure & Appl. Chem*, 1976, vol. 45, 11-30
- **FOSSIEZ et al.** *J Exp Med.*, 1996, vol. 183 (6), 2593-603
- Binding site elucidation and structure guided design of macrocyclic IL-17A antagonists. *Scientific Reports*, 2016, vol. 6
- **LIU, S et al.** Binding site elucidation and structure guided design of macrocyclic IL-17A antagonists. *Scientific Reports*, 2016, vol. 6, 30859
- **LIU S et al.** Binding site elucidation and structure guided design of macrocyclic IL-17A antagonists. *Sci Rep*, August 2016, vol. 16 (6), 30859
- **WADSWORTH SA** ; **CAVENDER DE** ; **BEERS SA** ; **LALAN P** ; **SCHAFER PH** ; **MALLOY EA et al.** RWJ 67657, a potent, orally active inhibitor of p38 mitogen-activated protein kinase. *Pharmacol Exp Ther*, 1999, vol. 291, 680-687
- **MURRAY BW et al.** Small-molecule p21-activated kinase inhibitor PF-3758309 is a potent inhibitor of oncogenic signaling and tumor growth. *Proc Natl Acad Sci U S A.*, 18 May 2010, vol. 107 (20), 9446-51